# EUROPEAN PATENT SPECIFICATION

(11) **EP 3 763 364 B1**
(45) Date of publication and mention of the grant of the patent: **05.11.2025**
(21) Application number: 20190189.9
(22) Date of filing: 17.07.2014
(51) Int. Cl.: A61K 9/48, A61K 9/107, A61K 47/26, A61K 47/10, A61K 47/24, A61K 31/202, A61K 31/232, A61P 1/16, A61P 3/06, A61P 7/02, A61P 7/04, A61P 9/00, A61P 9/10, A61P 25/00, A61P 25/24, A61P 25/28, A61P 29/00, A61P 35/00

(54) **SELF-EMULSIFYING COMPOSITION OF OMEGA-3 FATTY ACID**
SELBSTEMULGIERENDE OMEGA-3-FETTSÄUREZUSAMMENSETZUNG
COMPOSITION AUTO-ÉMULSIFIANTE D'ACIDE GRAS OMÉGA-3

(30) Priority: 18.07.2013 JP 2013149662
(43) Date of publication of application: 13.01.2021
(62) Divisional of application: 14826603.4
(73) Proprietor: Mochida Pharmaceutical Co., Ltd., Tokyo 160-8515 (JP)
(72) Inventor: ITO, Hiromitsu, Shinjuku-ku, Tokyo 160-8515 (JP); FUJII, Hirosato, Shinjuku-ku, Tokyo 160-8515 (JP); YAMAGATA, Motoo, Shinjuku-ku, Tokyo 160-8515 (JP)
(74) Representative: Grünecker Patent- und Rechtsanwälte PartG mbB

(56) References cited:
- EP-A1- 2 433 630
- WO-A1-2010/103402

## Description

### TECHNICAL FIELD

This invention provides a self-emulsifying composition containing at least one member selected from the group consisting of w3 polyunsaturated fatty acids and their pharmaceutically acceptable salts and esters. The specification also discloses a pharmaceutical product and its production method.

### BACKGROUND ART

Known ω3 polyunsaturated fatty acids (hereinafter abbreviated as w3 PUFA) include α-linolenic acid, eicosapentaenoic acid (hereinafter abbreviated as EPA), and docosahexaenoic acid (hereinafter abbreviated as DHA). Since the ω3 PUFA and pharmaceutically acceptable salts and esters thereof have actions such as anti-arteriosclerosis action, platelet aggregation suppressive action, blood lipid lowering action, anti-inflammatory action, carcinostatic action, and central action, they are blended in various food products, and commercially sold in the form of health food and medical and pharmaceutical products.

Ethyl eicosapentaenoate ester (hereinafter abbreviated as EPA-E) is commercially sold in Japan as an oral therapeutic agent for ameliorating ulcer, pain, and coldness associated with arteriosclerosis obliterans as well as hyperlipidemia (product name Epadel, Mochida Pharmaceutical Co., Ltd.). When EPA-E is orally administered under fasting, increase in plasma EPA concentration is smaller than the case of the oral administration after the meal conceivably because absorption of the EPA-E requires secretion of bile acid and food components as a carrier. Accordingly, Epadel is instructed to be orally administered immediately after the meal (see Non-Patent Literature 1).

However, dosage method or drug compliance has become a problem for those people not taking breakfast with the recent change in the life style, patients who can only take meals at a reduced amount, patients who can only take a fluid diet (milk, rice broth, starch gruel, egg, soup, juice, or oral nutritional supplement), patients with reduced absorption ability of the intestinal tract (for example, elderly, patients of intestinal disease, patients after intestinal surgery, terminal cancer patients, and patients taking a lipase inhibitor), or patients who are unable to take meals such as those after the cerebral infarction.

Furthermore, the relation between coronary artery diseases and nonfasting hypertriglyceridemia having a normal serum triglyceride (hereinafter abbreviated as "TG") level under fasting but an abnormally-increased TG level after the meal, which may be prolonged, is drawing attention and therefore, there is a demand for an ω3 PUFA preparation that can be absorbed rapidly even if taken before the meal and suppress increase of serum TG after the meal.

As a self-emulsifying preparation which does not contain water in the preparation and which is readily dispersible and self-emulsifying when brought in contact with water, a self-emulsifying composition containing an ω3 PUFA and fenofibrate as its effective components, ethanol, and a surfactant has been reported (see Patent Literature 1 and Non-Patent Literature 4).

These compositions contain ethanol as a component added for improving the dissolution of the fenofibrate. However, volatilization of the ethanol is associated with the risk of capsule deformation and bubble inclusion in the capsule, damages in the quality such as capsule deformation and cracks, as well as denaturing of the content in the capsule such as cloudiness and separation. In addition, use of a preparation containing such composition should be difficult if not impossible for patients intolerable for the alcohol (ethanol).

A self-emulsifying composition containing ethanol and polyhydric alcohols in addition to the w3 PUFA and a surfactant which is capable of forming a dispersion having a small or very small average particle size when brought in contact with water is also reported (Patent Literature 2).

With regard to self-emulsifying compositions having a low ethanol content, a self-emulsifying composition comprising an ω3 PUFA, an emulsifier having a hydrophile lipophile balance (hereinafter abbreviated as HLB) of at least 10, lecithin, and a polyhydric alcohol such as propylene glycol or glycerin which has high self-emulsifying property, oral fasting absorbability and absorption speed has been reported (Patent Literature 3).

When a composition containing a co-solvent such as a polyhydric alcohol is accommodated in a capsule, the co-solvent moves to the capsule film to cause denaturing of the composition as well as capsule deformation due to the softening of the capsule (Patent Literature 4).

Self-emulsifying compositions, as generally containing larger amounts of emulsifiers and, accordingly, being increased in total amount, are liable to cause inflammation of the gastrointestinal tract or have a reduced content per capsule of the biologically active component dissolved in oil component (Patent Literature 5). Accordingly, the emulsifier used in the self-emulsifying composition is preferably the one which is non-toxic or less-toxic even in the case of continuous administration, and the emulsifier is preferably used at a low content.

In view of compliance, amount of the emulsifier and alcohols incorporated should be minimized also in consideration of reducing the size of the preparation because amount of the preparation that has to be taken per administration increases with the increase in the amount of the components other than the ω3 PUFA in the self-emulsifying composition since predetermined amount of the ω3 PUFA should be taken per administration.
EP2433630 (A1) discloses a self-emulsifying composition of ω3 fatty acid.

### CITATION LIST

### PATENT LITERATURES

Patent Literature 1: JP 2008-516890 A
Patent Literature 2: JP 2012-519728 A
Patent Literature 3: WO 2010/134614
Patent Literature 4: JP 2011-12003 A
Patent Literature 5: JP 2012-180337 A

### NON-PATENT LITERATURES

Non-Patent Literature 1: Epadel S (Drug Interview Form), Mochida Pharmaceutical Co., Ltd., June, 2012
Non-Patent Literature 2: "Guideline for Diagnosis and Prevention of Atherosclerotic Cardiovascular Diseases, 2007 Edition" edited by Japan Atherosclerosis Society and published by Kyowa Kikaku Ltd., April 25, 2007
Non-Patent Literature 3: Diabetes, vol. 57, no. 9, 2382-2392, 2008
Non-Patent Literature 4: European Journal of Pharmaceutical Sciences, vol. 33, 351-360, 2008
Non-Patent Literature 5: "2007 Dictionary of Drug Additives" edited by International Pharmaceutical Excipients Council Japan and published by Yakuji Nippo Ltd., July 25, 2007

### SUMMARY OF INVENTION

### TECHNICAL PROBLEMS

There is a demand for a preparation wherein ethanol and polyhydric alcohols added to the self-emulsifying composition have been reduced.

There is also a demand for a preparation wherein emulsifier added to the self-emulsifying composition has been reduced.

There is also a demand for a preparation wherein content of the ω3 PUFA in the self-emulsifying composition has been increased.

There is also a demand for a self-emulsifying composition which shows excellent drug compliance.

There is also a demand for a self-emulsifying composition which is free from denaturing such as cloudiness and separation and which retains the good appearance during its storage at room temperature, and also, in low temperature and high temperature environments since use of the self-emulsifying composition as a drug may involve storage in cold district and other environments.

There is also a demand for a self-emulsifying composition wherein the composition has stable quality.

There is also a demand for a preparation wherein the self-emulsifying composition has been encapsulated.

There is also a demand for a preparation wherein softening of the capsule film after the encapsulation of the self-emulsifying composition has been suppressed so that the encapsulated preparation is not deformed.

Accordingly, an object of the present invention is to provide a self-emulsifying composition which has realized one or more of the demands as described above. Another object of the present invention is to provide a preparation encapsulating such composition.

### SOLUTION TO PROBLEMS

The subject matter of the invention is as set out in the appended claims.

In view of the problems as described above, the inventors of the present invention made an intensive investigation on the components which would be substitutes for the ethanol and the polyhydric alcohols, and found that a predetermined amount of water is useful in improving the compatibility of the self-emulsifying composition.

The inventors found that the water content in the composition can be as low as 0.5 to 6% by weight which is a content lower than the ethanol or the polyhydric alcohol, and the amount of the emulsifier agent can be further reduced by the compatibilizing effect of this small amount of the water, and accordingly, a self-emulsifying composition having a high content of the ω3 PUFA is thereby produced.

The inventors also found that a self-emulsifying composition which exhibits excellent properties with regard to at least one of the problems as described above can be produced by such composition. The present invention has been completed on the basis of such findings.

The inventors also found that the content of the emulsifier can be further reduced, and a self-emulsifying composition having a high content of the ω3 PUFA was thereby completed. The present invention has been completed also on the basis of such findings.

The composition of the present invention is a composition which exhibits excellent properties with regard to at least one of the problems as described above.

One aspect of the invention relates to a self-emulsifying composition comprising, when the total amount of the self-emulsifying composition is 100% by weight,
a) 70 to 90% by weight of at least one compound selected from the group consisting of an ω3 PUFA, its pharmaceutically acceptable salt, and its ester,
b) 0.5 to 6% by weight of water,
c) 1 to 29% by weight of an emulsifier which is a polyoxyethylene sorbitan fatty acid ester, and
d) 3 to 25 parts by weight of lecithin in relation to 100 parts by weight of the ω3 polyunsaturated fatty acid, its pharmaceutically acceptable salt, and its ester,
   wherein
e) content of ethanol is up to 4% by weight of total amount of the composition, and
f) content of polyhydric alcohol is up to 4% by weight of total amount of the composition.

Preferred embodiments are set forth in the dependent claims.

The references to methods of treatment in the subsequent paragraphs of this description are to be interpreted as references to the compounds, pharmaceutical compositions and medicaments of the present invention for use in a method for treatment of the human (or animal) body by therapy.

The following embodiments of the "first aspect of the invention" are not according to the invention unless they are embraced by the claims.
Accordingly, the first aspect of the present invention is the self-emulsifying composition as described below.
(1-1) A self-emulsifying composition comprising, when the total amount of the self-emulsifying composition is 100% by weight,
   a) 70 to 90% by weight of at least one compound selected from the group consisting of an ω3 PUFA, its pharmaceutically acceptable salt, and its ester,
   b) 0.5 to 6% by weight of water,
   c) 1 to 29% by weight of an emulsifier (excluding lecithin) which is preferably a polyoxyethylene sorbitan fatty acid ester, and
   d) 3 to 40 parts by weight of lecithin in relation to 100 parts by weight of the ω3 polyunsaturated fatty acid, its pharmaceutically acceptable salt, and its ester,
      wherein
   e) content of ethanol and/or polyhydric alcohol is up to 4% by weight of total amount of the composition.
(1-2) A self-emulsifying composition comprising, when the total amount of the self-emulsifying composition is 100% by weight,
   a) 70 to 90% by weight of at least one compound selected from the group consisting of an w3 PUFA, its pharmaceutically acceptable salt, and its ester,
   b) 0.5 to 6% by weight of water,
   c) 1 to 29% by weight of an emulsifier which is a polyoxyethylene sorbitan fatty acid ester, and
   d) 3 to 40 parts by weight of lecithin in relation to 100 parts by weight of at least one compound selected from the group consisting of the ω3 polyunsaturated fatty acid, its pharmaceutically acceptable salt, and its ester,
      wherein
   e) content of ethanol is up to 4% by weight of total amount of the composition, and
   f) content of polyhydric alcohol is up to 4% by weight of total amount of the composition.
(1-3) The self-emulsifying composition according to (1-1) or (1-2) wherein the polyoxyethylene sorbitan fatty acid ester is at least one member selected from the group consisting of polyoxyethylene (20) sorbitan monolaurate, polyoxyethylene (20) sorbitan monopalmitate, polyoxyethylene (20) sorbitan monostearate, polyoxyethylene (20) sorbitan tristearate, polyoxyethylene (20) sorbitan monoisostearate, polyoxyethylene (20) sorbitan monooleate, and polyoxyethylene (20) sorbitan trioleate.
(1-4) The self-emulsifying composition according to any one of (1-1) to (1-3) wherein the emulsifier further includes a polyoxyethylene hydrogenated castor oil and/or a polyoxyethylene castor oil.
(1-5) The self-emulsifying composition according to any one of (1-1) to (1-4) wherein the emulsifier further includes a polyoxyethylene castor oil.
(1-6) A self-emulsifying composition according to any one of (1-1) to (1-5) wherein the polyhydric alcohol is propylene glycol or glycerin.
(1-7) A self-emulsifying composition according to any one of (1-1) to (1-6) wherein the composition contains 0 to 4% by weight of a polyhydric alcohol.
(1-8) A self-emulsifying composition according to any one of (1-1) to (1-6) wherein the composition does not contain more than 4% by weight of the polyhydric alcohol.
(1-9) A self-emulsifying composition according to any one of (1-1) to (1-8) wherein the content of the polyhydric alcohol in the composition is up to 1% by weight.
(1-10) A self-emulsifying composition according to any one of (1-1) to (1-9) wherein content of the polyhydric alcohol in the composition is 0 to 1% by weight.
(1-11) A self-emulsifying composition according to any one of (1-1) to (1-8) wherein the composition does not contain more than 1% by weight of the polyhydric alcohol.
(1-12) A self-emulsifying composition according to any one of (1-1) to (1-11) wherein the composition does not substantially contain polyhydric alcohol.
(1-13) A self-emulsifying composition according to any one of (1-1) to (1-12) wherein content of the ethanol in the composition is up to 4% by weight.
(1-14) A self-emulsifying composition according to any one of (1-1) to (1-12) wherein content of the ethanol in the composition is 0 to 4% by weight.
(1-15) A self-emulsifying composition according to any one of (1-1) to (1-12) wherein the composition does not contain more than 4% by weight of the ethanol.
(1-16) A self-emulsifying composition according to any one of (1-1) to (1-15) wherein the composition does not substantially contain the ethanol.
(1-17) A self-emulsifying composition according to any one of (1-1) to (1-16) wherein the ω3 PUFA, its pharmaceutically acceptable salt, or its ester is at least one member selected from the group consisting of EPA, DHA, and their pharmaceutically acceptable salts and esters.
(1-18) A self-emulsifying composition according to any one of (1-1) to (1-17) wherein the ester of the ω3 PUFA is ethyl ester or triglyceride ester.
(1-19) A self-emulsifying composition according to any one of (1-1) to (1-18) wherein the ω3 PUFA, its pharmaceutically acceptable salt, or its ester is EPA-E or ethyl DHA ester (hereinafter abbreviated as DHA-E).
(1-20) A self-emulsifying composition according to any one of (1-1) to (1-19) wherein the composition contains at least one member selected from the group consisting of EPA, DHA, and their pharmaceutically acceptable salts and esters as its effective component.
(1-21) A self-emulsifying composition according to any one of (1-1) to (1-20) wherein the composition contains EPA-E and/or DHA-E as its effective component.
(1-22) A self-emulsifying composition according to any one of (1-1) to (1-21) wherein the composition contains EPA-E as its effective component.
(1-23) A self-emulsifying composition according to any one of (1-1) to (1-22) wherein the lecithin is at least one member selected from the group consisting of soybean lecithin, zymolytic soybean lecithin, hydrogenated soybean lecithin, and egg yolk lecithin.
(1-24) A self-emulsifying composition according to any one of (1-1) to (1-23) wherein content of at least one emulsifier selected from the group consisting of polyoxyethylene hydrogenated castor oil, polyethylene glycol fatty acid ester, and polyoxyethylene polyoxypropylene glycol is less than 5% by weight of the total composition. In addition, a self-emulsifying composition according to any one of (1-1) to (1-23) wherein content of each emulsifier including polyoxyethylene hydrogenated castor oil, polyoxyethylene glycol fatty acid ester, or polyoxyethylene polyoxypropylene glycol is less than 5% by weight of the total composition.
(1-25) A self-emulsifying composition according to any one of (1-1) to (1-24) wherein a) to d) have been mixed in arbitrary order.
(1-26) A self-emulsifying composition according to any one of (1-1) to (1-25) wherein the composition has a transparent appearance when the composition is allowed to stand.
(1-27) A self-emulsifying composition according to any one of (1-1) to (1-25) wherein the composition has a nonseparated or non-cloudy appearance when the composition is allowed to stand.
(1-28) A self-emulsifying composition according to any one of (1-1) to (1-27) wherein the composition has a transparent appearance when the composition is stored in the environment of 5°C or 40°C for 12 hours.
(1-29) A self-emulsifying composition according to any one of (1-1) to (1-28) wherein the composition has a nonseparated or non-cloudy appearance when the composition is stored in the environment of 5°C or 40°C for 12 hours.
(1-30) A self-emulsifying composition according to any one of (1-1) to (1-29) wherein the composition is excellent in at least one of self-emulsifying property, composition dispersibility, and emulsion stability.
(1-31) A self-emulsifying composition according to any one of (1-1) to (1-30) wherein the composition spontaneously emulsifies when 10 µL of the composition is added dropwise to 5 mL of purified water or first solution in the dissolution test of Japanese Pharmacopeia at 37°C.
(1-32) A self-emulsifying composition according to any one of (1-1) to (1-31) wherein the composition disperses when 10 µL of the composition is added dropwise to 5 mL of purified water or first solution in the dissolution test of Japanese Pharmacopeia at 37°C and the mixture is stirred.
(1-33) A self-emulsifying composition according to any one of (1-1) to (1-32) wherein separation of the oil component does not occur when 10 µL of the composition is added dropwise to 5 mL of purified water or first solution in the dissolution test of Japanese Pharmacopeia at 37°C.
(1-34) A self-emulsifying composition according to any one of (1-1) to (1-33) wherein, when the self-emulsifying composition is orally administered to a male beagle which has been fasted for at least 18 hours at an amount corresponding to 600 mg of the at least one compound selected from the group consisting of ω3 PUFA, its pharmaceutically acceptable salts, and its esters, maximum blood ω3 PUFA concentration (corrected by subtracting the blood ω3 concentration before the administration of the composition) is at least 50 µg/mL, and/or area under the blood ω3 PUFA concentration vs time curve from the administration to two hours after the administration is at least 30 µg/mL.hr; maximum blood ω3 PUFA concentration is at least 50 µg/mL, and/or area under the blood ω3 PUFA concentration vs time curve from the administration to two hours after the administration is at least 50 µg/mL.hr; maximum blood ω3 PUFA concentration is at least 60 µg/mL, and/or area under the blood ω3 PUFA concentration vs time curve from the administration to two hours after the administration is at least 60 µg/mL.hr; or maximum blood ω3 PUFA concentration is at least 70 µg/mL, and/or area under the blood ω3 PUFA concentration vs time curve from the administration to two hours after the administration is at least 70 µg/mL.hr.
(1-35) A self-emulsifying composition according to any one of (1-1) to (1-33) wherein, when the self-emulsifying composition according to any one of (1-1) to (1-33) is orally administered to a male cynomolgus monkey (Macaca fascicularis) which has been fasted for at least 12 hours at an amount corresponding to 45 mg/kg body weight of the at least one compound selected from the group consisting of ω3 PUFA, its pharmaceutically acceptable salts, and its esters, maximum blood w3 PUFA concentration (corrected by subtracting the blood ω3 concentration before the administration of the composition) is at least 50 µg/mL, and/or area under the blood ω3 PUFA concentration vs time curve from the administration to 12 hours after the administration is at least 400 µg/mL.hr; or maximum blood ω3 PUFA concentration is at least 70 µg/mL, and/or area under the blood ω3 PUFA concentration vs time curve from the administration to 12 hours after the administration is at least 500 µg/mL.hr.
(1-36) Use of the self-emulsifying composition according to any one of (1-1) to (1-33) wherein, when the self-emulsifying composition according to any one of (1-1) to (1-33) is orally administered to a human before meals at an amount corresponding to 1800 mg of the at least one compound selected from the group consisting of ω3 PUFA, its pharmaceutically acceptable salts, and its esters, maximum blood ω3 PUFA concentration (corrected by subtracting the blood ω3 concentration before the administration of the composition) is at least 50 µg/mL, and/or the blood ω3 PUFA concentration two hours after the administration is at least 10 µg/mL.
(1-37) A self-emulsifying composition according to any one of (1-1) to (1-33) wherein, when the self-emulsifying composition according to any one of (1-1) to (1-33) is orally administered to a human before meals at an amount corresponding to 1800 mg of the at least one compound selected from the group consisting of ω3 PUFA, its pharmaceutically acceptable salts, and its esters, maximum blood ω3 PUFA concentration (corrected by subtracting the blood ω3 concentration before the administration of the composition) is at least 10 µg/mL, and/or area under the blood ω3 PUFA concentration vs time curve from the administration to 72 hours after the administration is at least 250 µg/mL.hr.
(1-38) A self-emulsifying composition according to any one of (1-1) to (1-37) wherein content of polyoxyethylene castor oil is up to 120 parts by weight in relation to 100 parts by weight of a polyoxyethylene sorbitan fatty acid ester in the composition.
(1-39) A self-emulsifying composition comprising, when the total amount of the self-emulsifying composition is 100% by weight,
   a) 70 to 90% by weight of EPA-E,
   b) 0.5 to 6% by weight of water,
   c) 1 to 29% by weight of an emulsifier which is a polyoxyethylene sorbitan fatty acid ester, and
   d) 3 to 40 parts by weight of lecithin in relation to 100 parts by weight of the EPA-E,
      wherein
   e) content of ethanol and/or polyhydric alcohol is up to 4% by weight of total amount of the composition.
(1-40) A self-emulsifying composition comprising, when the total amount of the self-emulsifying composition is 100% by weight,
   a) 70 to 90% by weight of EPA-E,
   b) 0.5 to 6% by weight of water,
   c) 1 to 29% by weight of an emulsifier which is a polyoxyethylene sorbitan fatty acid ester, and
   d) 3 to 40 parts by weight of lecithin in relation to 100 parts by weight of the EPA-E,
      wherein
   e) content of ethanol is up to 4% by weight of total amount of the composition, and
   f) content of polyhydric alcohol is up to 4% by weight of total amount of the composition.

The present specification further discloses a second aspect, said second aspect being an encapsulated self-emulsifying preparation as described below.
(2-1) A self-emulsifying preparation retaining the self-emulsifying composition according to any one of (1-1) to (1-40), wherein the self-emulsifying composition is encapsulated in a hard capsule and/or a soft capsule.
(2-2) A self-emulsifying preparation according to (2-1) which exhibits sufficient hardness immediately after its production.
(2-3) A self-emulsifying preparation according to (2-1) or (2-2) wherein the preparation has a hardness of 177N (18 kgf) or more immediately after its production.
(2-4) A self-emulsifying preparation according to any one of (2-1) to (2-3) wherein the preparation does not experience loss of its hardness of 59 N (6 kgf)or more when the preparation sealed in an aluminum package is stored at 40°C for 1 week and compared with the preparation before the storage.
(2-5) A self-emulsifying preparation according to any one of (2-1) to (2-4) wherein the preparation has a hardness of 196 N (20 kgf) or more when the preparation sealed in an aluminum package is stored at 40°C for 1 week.
(2-6) A self-emulsifying preparation according to any one of (2-1) to (2-5) wherein, when the preparation sealed in an aluminum package is stored at 40°C for 1 week, the preparation retains 60% or more of its hardness before the storage.
(2-7) A preparation according to (2-1) which is at least one selected from the group consisting of therapeutic agent for dyslipidemias (hypercholesterolemia, high LDL cholesterolemia, high non-HDL cholesterolemia, high VLDL cholesterolemia, low HDL cholesterolemia, hypertriglyceridemia, hyperapobetalipoproteinemia, apo A-I hypolipoproteinemia, and the like), therapeutic agent for postprandial hypertriglyceridemia, anti-arteriosclerosis agent, platelet aggregation suppressant, therapeutic agent for peripheral circulatory insufficiency, agent for preventing occurrence of cardiovascular events, therapeutic agent for inflammatory diseases (non-alcoholic fatty liver disease (hereinafter abbreviated as NAFLD), non-alcoholic steatohepatitis (hereinafter abbreviated as NASH), and the like), progression inhibitor and therapeutic agent for dementia (Alzheimer-type dementia, cerebrovascular dementia, mixed type dementia, and the like), anticancer agent, and therapeutic agent for central diseases (clinical depression, depressive state, obsessive-compulsive disorder, social anxiety disorder, panic disorder, and the like).

The specification further discloses a third aspect, said third aspect being a method for producing the self-emulsifying composition as described below.
(3-1) A method for producing the self-emulsifying composition comprising the step of mixing the following components a) to d) in any order, while the total amount of the self-emulsifying composition is 100% by weight,
   a) 70 to 90% by weight of at least one compound selected from the group consisting of ω3 PUFA, its pharmaceutically acceptable salts, and its esters,
   b) 0.5 to 6% by weight of water,
   c) 1 to 29% by weight of an emulsifier which is an polyoxyethylene sorbitan fatty acid ester, and
   d) 3 to 40 parts by weight of lecithin in relation to 100 parts by weight of at least one compound selected from the group consisting of ω3 polyunsaturated fatty acid, its pharmaceutically acceptable salts, and its esters,
      so that
   e) content of ethanol and/or polyhydric alcohol is up to 4% by weight of total amount of the composition.
(3-2) A method for producing the self-emulsifying composition comprising the step of mixing the following components a) to d) in any order, while the total amount of the self-emulsifying composition is 100% by weight,
   a) 70 to 90% by weight of at least one compound selected from the group consisting of ω3 PUFA, its pharmaceutically acceptable salts, and its esters,
   b) 0.5 to 6% by weight of water,
   c) 1 to 29% by weight of an emulsifier which is an polyoxyethylene sorbitan fatty acid ester, and
   d) 3 to 40 parts by weight of lecithin in relation to 100 parts by weight of at least one compound selected from the group consisting of ω3 polyunsaturated fatty acid, its pharmaceutically acceptable salts, and its esters,
      so that
   e) content of ethanol is up to 4% by weight of total amount of the composition, and
   f) content of polyhydric alcohol is up to 4% by weight of total amount of the composition.
(3-3) A method for producing the self-emulsifying composition according to (3-1) or (3-2) further including the step of mixing the a), b), and/or c) by heating at a temperature of 70°C or higher.

The specification further discloses a fourth aspect, said fourth aspect being a pharmaceutical preparation as described below for administering the self-emulsifying composition by a particular method.
(4-1) A preparation for orally administering the self-emulsifying composition or the encapsulated self-emulsifying preparation according to any one of the above (1-1) to (1-40) and (2-1) to (2-7) as a drug or a veterinary drug under fasting or at bedtime.
(4-2) A preparation for orally administering the self-emulsifying composition or the encapsulated self-emulsifying preparation produced by the method according to any one of the above (3-1) to (3-3) as a drug or a veterinary drug under fasting or at bedtime.
(4-3) A preparation according to (4-1) or (4-2) which is at least one selected from the group consisting of therapeutic agent for dyslipidemias (hypercholesterolemia, high LDL cholesterolemia, high non-HDL cholesterolemia, high VLDL cholesterolemia, low HDL cholesterolemia, hypertriglyceridemia, hyperapobetalipoproteinemia, apo A-I hypolipoproteinemia, and the like), therapeutic agent for postprandial hypertriglyceridemia, anti-arteriosclerosis agent, platelet aggregation suppressant, therapeutic agent for peripheral circulatory insufficiency, agent for preventing occurrence of cardiovascular events, therapeutic agent for inflammatory diseases (NAFLD, NASH, and the like), anticancer agent, and preventive agent, therapeutic agent and progression inhibitor for central diseases (clinical depression, depressive state, obsessive-compulsive disorder, social anxiety disorder, panic disorder, and the like).
(4-4) A preparation according to any one of the above (4-1) to (4-3) which is administered once daily.
(4-5) A method for administering and/or using the preparation according to any one of the above (4-1) to (4-4).
(4-6) A method for increasing the concentration of ω3 PUFA in plasma by the oral administration according to any one of the above (4-1) to (4-4).

The specification further discloses a fifth aspect, said fifth aspect being a prophylactic, progression inhibiting, or therapeutic method for at least one disease selected from the group described below.
(5-1) Prophylactic, progression inhibiting, and treatment methods for at least one disease selected from the group consisting of dyslipidemias (hypercholesterolemia, high LDL cholesterolemia, high non-HDL cholesterolemia, high VLDL cholesterolemia, low HDL cholesterolemia, hypertriglyceridemia, hyperapobetalipoproteinemia, apo A-I hypolipoproteinemia, and the like), postprandial hypertriglyceridemia, arteriosclerosis, platelet hyperaggregation, peripheral circulatory insufficiency, occurrence of cardiovascular events, inflammatory diseases (NAFLD, NASH, and the like), dementia (Alzheimer-type dementia, cerebrovascular dementia, mixed type dementia, and the like), cancer, and central diseases (clinical depression, depressive state, obsessive-compulsive disorder, social anxiety disorder, panic disorder, and the like), wherein at least one selected from the self-emulsifying compositions and the encapsulated self-emulsifying preparations according to (1-1) to (1-40), (2-1) to (2-7), and (3-1) to (3-3) is orally administered to a patient as a drug or a veterinary drug.
(5-2) Methods according to (5-1) wherein the self-emulsifying composition or the encapsulated self-emulsifying preparation is orally administered as a drug or a veterinary drug under fasting or at bedtime.
(5-3) A method according to (5-1) or (5-2) wherein the self-emulsifying composition or the encapsulated self-emulsifying preparation is administered as a drug or a veterinary drug once daily.

The specification further discloses a sixth aspect, said sixth aspect being a self-emulsifying composition as described below.
(6-1) A self-emulsifying composition wherein, when the self-emulsifying composition is orally administered to a male beagle which has been fasted for at least 18 hours at an amount corresponding to 600 mg of at least one compound selected from the group consisting of ω3 PUFA, its pharmaceutically acceptable salts, and its esters, maximum blood ω3 PUFA concentration (corrected by subtracting the blood ω3 concentration before the administration of the composition) is at least 50 µg/mL, and/or area under the blood ω3 PUFA concentration vs time curve from the administration to two hours after the administration is at least 30 µg/mL.hr; maximum blood ω3 PUFA concentration is at least 50 µg/mL, and/or area under the blood ω3 PUFA concentration vs time curve from the administration to two hours after the administration is at least 50 µg/mL.hr; maximum blood ω3 PUFA concentration is at least 60 µg/mL, and/or area under the blood ω3 PUFA concentration vs time curve from the administration to two hours after the administration is at least 60 µg/mL.hr; or maximum blood ω3 PUFA concentration is at least 70 µg/mL, and/or area under the blood w3 PUFA concentration vs time curve from the administration to two hours after the administration is at least 70 µg/mL.hr.
(6-2) A self-emulsifying composition wherein, when the self-emulsifying composition is orally administered to a male cynomolgus monkey which has been fasted for at least 12 hours at an amount corresponding to 45 mg/kg body weight of at least one compound selected from the group consisting of ω3 PUFA, its pharmaceutically acceptable salts, and its esters, maximum blood w3 PUFA concentration (corrected by subtracting the blood ω3 concentration before the administration of the composition) is at least 50 µg/mL, and/or area under the blood ω3 PUFA concentration vs time curve from the administration to 12 hours after the administration is at least 400 µg/mL.hr; or maximum blood ω3 PUFA concentration is at least 70 µg/mL, and/or area under the blood ω3 PUFA concentration vs time curve from the administration to 12 hours after the administration is at least 500 µg/mL.hr.
(6-3) A self-emulsifying composition wherein, when the self-emulsifying composition is orally administered to a human before meals at an amount corresponding to 1800 mg of at least one compound selected from the group consisting of ω3 PUFA, its pharmaceutically acceptable salts, and its esters, maximum blood ω3 PUFA concentration (corrected by subtracting the blood ω3 concentration before the administration of the composition) is at least 50 µg/mL, and/or the blood ω3 PUFA concentration two hours after the administration is at least 10 µg/mL.
(6-4) A self-emulsifying composition wherein, when the self-emulsifying composition is orally administered to a human before meals at an amount corresponding to 1800 mg of at least one compound selected from the group consisting of ω3 PUFA, its pharmaceutically acceptable salts, and its esters, maximum blood ω3 PUFA concentration (corrected by subtracting the blood ω3 concentration before the administration of the composition) is at least 10 µg/mL, and/or area under the blood ω3 PUFA concentration vs time curve from the administration to 72 hours after the administration is at least 250 µg/mL.hr.

### ADVANTAGEOUS EFFECTS OF INVENTION

The self-emulsifying composition of the present invention contains a small amount of water instead of the ethanol and the polyhydric alcohol in its composition. Compatibility of the composition improves by such composition, and amount of the emulsifier used can also be reduced, and accordingly, safety for animals (including human) is thereby improved. In addition, the ω3 PUFA will be included at a higher content, and this enables reduction in the amount of emulsifier used, and compliance is thereby improved.

Inclusion of the water in the composition also enables a composition without or with minimized use of the ethanol or the polyhydric alcohols, and hence, prevention of the softening of the capsule film, and deformation of the capsule.

The self-emulsifying composition of the present invention is excellent at least in one of compatibility (appearance), self-emulsifying property, dispersibility of the composition, emulsion stability, and absorbability, and it will be rapidly absorbed even if it is administered before the meal or after ingestion of the low-fat meal to suppress increase in the serum TG after the meal, or it is administered at bedtime to prevent deficiency of essential fatty acid at the time of taking a lipase inhibitor.

The self-emulsifying composition enables not only the storage at room temperature but also the storage under the conditions of low temperature (for example, 5°C) and high temperature (for example, 40°C) without causing separation or cloudiness of the composition, namely, with good appearance.

The self-emulsifying composition of the present invention has at least one, preferably at least two, and more preferably all of the advantageous characters as described above.

### DESCRIPTION OF EMBODIMENTS

The present invention is described below in detail.

The present invention relates to a self-emulsifying composition comprising 70 to 90% by weight in total of at least one compound selected from the group consisting of ω3 PUFA, its pharmaceutically acceptable salts, and its esters, 1 to 29% by weight of a particular emulsifier as defined in claim 1, and 3 to 25 parts by weight of lecithin in relation to 100 parts by weight of the ω3 PUFA, its pharmaceutically acceptable salt, or ester, wherein the composition has low or no content of ethanol or polyhydric alcohol. The present specification also discloses discloses a self-emulsifying preparation having such self-emulsifying composition encapsulated therein, and a pharmaceutical product, a production method, and a method of use thereof.

In the present invention, "ω3 PUFA" is a fatty acid having a plurality of carbon - carbon double bonds in the molecule, and the first double bond is at 3rd position from the end on the side of the methyl group. Typical examples include α -linolenic acid, EPA, DHA, eicosatrienoic acid, stearidonic acid, eicosatetraenoic acid, clupanodonic acid, tetracosapentaenoic acid, and nisinic acid. Unless otherwise specified, the term "ω3 PUFA," "EPA," "DHA," and "fatty acid" as used in the present invention include not only the ω3 PUFA, EPA, DHA, and fatty acid but also pharmaceutically acceptable salts and esters thereof, respectively.

The ω3 PUFA used in the present invention may be a synthetic, semi-synthetic, natural ω3 PUFA, or a natural oil containing such ω3 PUFA. Examples of the natural ω3 PUFA include an extract from a natural oil containing an ω3 PUFA, a crudely purified natural oil containing an ω3 PUFA, and a highly purified natural oil containing an ω3 PUFA produced by a method known in the art. Exemplary semi-synthetic ω3 PUFAs include ω3 PUFAs produced by a microorganism or the like and the ω3 PUFAs or the natural ω3 PUFAs which have been subjected to a chemical treatment such as esterification or ester exchange. In the present invention, the ω3 PUFAs may be used alone or in combination of two or more.

In the present invention, EPA and DHA are the preferable examples of the ω3 PUFAs, and EPA is more preferable. Examples of the pharmaceutically acceptable salts of the ω3 PUFA include inorganic salts such as sodium salts and potassium salts, organic salts such as benzylamine salts and diethylamine salts, salts with basic amino acids such as arginine salts and lysine salts, and exemplary esters include alkyl esters such as ethyl ester, and esters such as monoglyceride, diglyceride and triglyceride (TG). Preferable examples include ethyl ester and TG ester, and the more preferred is ethyl ester. More specifically, preferable examples include EPA-E, TG ester of EPA, DHA-E, and TG ester of DHA, and among these, the more preferred are EPA-E and DHA-E, and the most preferred is EPA-E.

The ω3 PUFA used for the starting material of the self-emulsifying composition of the present invention is not particularly limited for its purity, and the purity is typically such that content of the ω3 PUFA in the total fatty acids of the composition of the present invention is preferably at least 50% by weight, more preferably at least 70% by weight, still more preferably at least 80% by weight, still more preferably at least 90% by weight, still more preferably at least 96.5% by weight, and most preferably at least 98% by weight. The w3 PUFA containing EPA at a high purity, for example, the one with the EPA content of at least 50% by weight in relation to the ω3 PUFA is preferable, and the content is more preferably at least 60% by weight, still more preferably at least 70% by weight, still more preferably at least 80% by weight, still more preferably at least 90% by weight, and most preferably at least 98% by weight. In other words, the composition of the present invention preferably has a high purity of ω3 PUFAs in the total fatty acid, more preferably, a high purity of EPA + DHA which are ω3 PUFAs, and most preferably, a high purity of EPA with substantially no DHA or a low content of DHA, for example, less than 1.0% by weight, preferably less than 0.5% by weight, more preferably less than 0.2% by weight of DHA.

For example, when EPA-E and DHA-E are used, compositional ratio of EPA-E / DHA-E and content of (EPA-E + DHA-E) in relation to total fatty acid are not particularly limited as long as the purity of EPA-E in the composition of the present invention is in the range as described above. However, the compositional ratio of the EPA-E / DHA-E is preferably at least 0.8, more preferably at least 1.0, and most preferably at least 1.2.

The composition of the present invention may also contain a polyunsaturated fatty acid other than the ω3 PUFA such as linoleic acid, y linolenic acid, or dihomo-γ-linolenic acid or pharmaceutically acceptable salt or ester thereof. However, content of arachidonic acid or pharmaceutically acceptable salt or ester thereof is preferably low, more preferably less than 2% by weight, still more preferably less than 1% by weight, and most preferably, the composition is substantially free from the arachidonic acid and pharmaceutically acceptable salt and ester thereof.

In the self-emulsifying composition of the present invention, content of the ω3 PUFA is 70 to 90% by weight, preferably 70 to 86% by weight, more preferably 72 to 85% by weight, and still more preferably 74 to 84% by weight. The ω3 PUFAs may be used alone or as a mixture of two or more. When a mixture of two or more ω3 PUFAs is used, the total amount of the mixture is 70 to 90% by weight in the self-emulsifying composition.

The ω3 PUFA used may be a soft capsule containing the EPA-E at a high purity (at least 96.5% by weight) (product name, Epadel; manufactured by Mochida Pharmaceutical Co., Ltd.) commercially available in Japan as a therapeutic agent for ASO and hyperlipidemia or a high purity EPA-E containing soft capsule (product name, VASCEPA; Amarin) commercially available in the U.S. as a therapeutic agent for hypertriglyceridemia. The ω3 PUFA used may also be a mixture of EPA-E and DHA-E, for example, Lovaza (Registered Trademark) (a soft capsule containing about 46.5% by weight of EPA-E and about 37.5% by weight of DHA-E from GlaxoSmithKline) commercially available in the U.S. as a therapeutic agent for hypertriglyceridemia or LOTRIGA (Registered Trademark) (a soft capsule containing about 46.5% by weight of EPA-E and about 37.5% by weight of DHA-E from Takeda Pharmaceutical Co., Ltd.) commercially available in Japan. A mixture of EPA and DHA used may also be, for example, Epanova (Registered Trademark) (a soft capsule containing about 50 to 60% by weight of EPA free acid and about 15 to 25% by weight of DHA free acid from AstraZeneca) commercially available in the U.S. as a therapeutic agent for hypertriglyceridemia.

Purified fish oils may also be used for the ω3 PUFA. Uses of monoglyceride, diglyceride, and TG derivatives and combinations thereof as the ω3 PUFA are also preferable embodiments. Various products containing the ω3 PUFA are commercially available, for example, Incromega F2250, F2628, E2251, F2573, TG2162, TG2779, TG2928, TG3525, and E5015 (Croda International PLC, Yorkshire, England), and EPAX6000FA, EPAX5000TG, EPAX4510TG, EPAX2050TG, EPAX7010EE, K85TG, K85EE, and K80EE (Pronova Biopharma, Lysaker, Norway). These products may be purchased and used for the composition of the present invention.

In the present invention, the "polyoxyethylene sorbitan fatty acid ester" is polyoxyethylene ether of a fatty acid ester wherein a part of the hydroxy groups of anhydrous sorbitol have been esterified with a fatty acid. Various compounds with different esterifying fatty acids are commercially available, and examples include polyoxyethylene (20) sorbitan monolaurate (NIKKOL TL-10, polysorbate 20, Tween 20), polyoxyethylene (20) sorbitan monopalmitate (NIKKOL TP-10V, Polysorbate 40, Tween 40), polyoxyethylene (20) sorbitan monostearate (NIKKOL TS-10MV, polysorbate 60, Tween 60), polyoxyethylene (20) sorbitan tristearate (NIKKOL TS-30V, polysorbate 65), polyoxyethylene (20) sorbitan monoisostearate (NIKKOL TI-10V), polyoxyethylene (20) sorbitan monooleate (NIKKOL TO-10MV, polysorbate 80, Tween 80), and polyoxyethylene (20) sorbitan trioleate (NIKKOL TO-30V, polysorbate 85), and the preferred are polyoxyethylene (20) sorbitan monooleate, polyoxyethylene (20) sorbitan monooleate, and polyoxyethylene (20) sorbitan trioleate, and the more preferred is polyoxyethylene (20) sorbitan monooleate.

These may be used alone or in combination of two or more. The term "polyoxyethylene sorbitan fatty acid ester" as used in the present invention means all of such compounds.

Content of the polyoxyethylene sorbitan fatty acid ester in the self-emulsifying composition of the present invention is as defined in claim 1. The content is 1 to 29% by weight, preferably 3 to 20% by weight, more preferably 5 to 15% by weight, and most preferably 5 to 9% by weight when the total amount of the self-emulsifying composition is 100% by weight.

In the present invention, the "polyoxyethylene castor oil" is a compound prepared by addition polymerization of ethylene oxide to castor oil. Various compounds with different average degrees of polymerization of ethylene oxide are commercially available. Examples thereof include NIKKOL CO-3 (Nikko Chemicals Co., Ltd.) with an average ethylene oxide mole number of 3, NIKKOL CO-10 (Nikko Chemicals Co., Ltd.) with an average ethylene oxide mole number of 10, EMALEX C-20 (Nippon Emulsion Co., Ltd.) with an average ethylene oxide mole number of 20, EMALEX C-30 (Nippon Emulsion Co., Ltd.) with an average ethylene oxide mole number of 30, Kolliphor EL (BASF) (polyoxyl 35 castor oil) with an average ethylene oxide mole number of 35, EMALEX C-40 (Nippon Emulsion Co.,Ltd.) with an average ethylene oxide mole number of 40, and EMALEX C-50 (Nippon Emulsion Co.,Ltd.) with an average ethylene oxide mole number of 50, and the preferred is Kolliphor EL. These may be used alone or in combination of two or more. The term "polyoxyethylene castor oil" as used in the present invention means all of such compounds unless otherwise noted.

Content of the polyoxyethylene castor oil in the self-emulsifying composition of the present invention is not particularly limited as long as the merits of the present invention are not adversely affected. The content is generally 1 to 20% by weight, preferably 2 to 15% by weight, more preferably 3 to 10% by weight, and most preferably 5 to 9% by weight when the total amount of the self-emulsifying composition is 100% by weight. Furthermore, the polyoxyethylene castor oil is preferably used at an amount such that the polyoxyethylene castor oil is used at up to 150 parts by weight, preferably up to 140 parts by weight, more preferably up to 130 parts by weight, still more preferably up to 120 parts by weight, particularly preferably up to 110 parts by weight, and most preferably up to 100 parts by weight in relation to 100 parts by weight of the polyoxyethylene sorbitan fatty acid ester in the composition. The quantity ratio of the polyoxyethylene sorbitan fatty acid ester to the polyoxyethylene castor oil contained in the composition is 100 parts by weight : 5 to 150 parts by weight, preferably 100 parts by weight : 10 to 140 parts by weight, more preferably 100 parts by weight : 20 to 130 parts by weight, still more preferably 100 parts by weight : 30 to 120 parts by weight, particularly preferably 100 parts by weight : 50 to 110 parts by weight, and most preferably 100 parts by weight : 80 to 120 parts by weight.

In the present invention, the "polyoxyethylene hydrogenated castor oil" is a compound prepared by addition polymerization of ethylene oxide to hydrogenated castor oil which is obtained by adding hydrogen to castor oil. Various compounds with different average degrees of polymerization of ethylene oxide are commercially available. Examples thereof include polyoxyethylene (20) hydrogenated castor oil (NIKKOL HCO-20, Nikko Chemicals Co., Ltd.), polyoxyethylene (40) hydrogenated castor oil (NIKKOL HCO-40, Nikko Chemicals Co., Ltd.), polyoxyethylene (50) hydrogenated castor oil (NIKKOL HCO-50, Nikko Chemicals Co., Ltd.), polyoxyethylene (60) hydrogenated castor oil (NIKKOL HCO-60, Nikko Chemicals Co., Ltd.), and polyoxyethylene (100) hydrogenated castor oil (NIKKOL HCO-100, Nikko Chemicals Co., Ltd.), and the preferred is polyoxyethylene (60) hydrogenated castor oil. These may be used alone or in combination of two or more. The term "polyoxyethylene hydrogenated castor oil" as used in the present invention means all of such compounds unless otherwise noted.

Content of the polyoxyethylene hydrogenated castor oil in the self-emulsifying composition of the present invention is not particularly limited as long as the merits of the present invention are not adversely affected. The content is generally 1 to 20% by weight, preferably 2 to 15% by weight, more preferably 3 to 10% by weight, and most preferably 5 to 9% by weight when the total amount of the self-emulsifying composition is 100% by weight. Furthermore, the polyoxyethylene hydrogenated castor oil is preferably used at an amount such that the polyoxyethylene hydrogenated castor oil is used at up to 150 parts by weight, preferably up to 140 parts by weight, more preferably up to 130 parts by weight, still more preferably up to 120 parts by weight, particularly preferably up to 110 parts by weight, and most preferably up to 100 parts by weight in relation to 100 parts by weight of the polyoxyethylene sorbitan fatty acid ester in the composition. The quantity ratio of the polyoxyethylene sorbitan fatty acid ester to the polyoxyethylene hydrogenated castor oil contained in the composition is 100 parts by weight : 5 to 150 parts by weight, preferably 100 parts by weight : 10 to 140 parts by weight, more preferably 100 parts by weight : 20 to 130 parts by weight, still more preferably 100 parts by weight : 30 to 120 parts by weight, particularly preferably 100 parts by weight : 50 to 110 parts by weight, and most preferably 100 parts by weight : 80 to 120 parts by weight.

The self-emulsifying composition of the present invention is characterized by its inclusion of a polyoxyethylene sorbitan fatty acid ester as the emulsifier. In a preferred embodiment of the present invention, a polyoxyethylene sorbitan fatty acid ester and polyoxyethylene castor oil and/or polyoxyethylene hydrogenated castor oil are included as the emulsifier. In another preferred embodiment of the present invention, a polyoxyethylene sorbitan fatty acid ester and polyoxyethylene castor oil are included as the emulsifier. The self-emulsifying composition of the present invention may also contain an emulsifier other than the polyoxyethylene sorbitan fatty acid ester and the polyoxyethylene castor oil, while content of such emulsifier is up to 20 parts by weight, more preferably up to 10 parts by weight, even more preferably less than 5 parts by weight, and most preferably substantially none when the total content of the emulsifier in the composition is 100 parts by weight. The additional emulsifier is not particularly limited as long as it satisfies at least one of the demands as described above, and exemplary additional emulsifiers include sorbitan fatty acid ester, glycerin fatty acid ester, polyoxyethylene hydrogenated castor oil, propylene glycol fatty acid ester, saturated polyglycolated glyceride, polyoxyethylene polyoxypropylene glycol, sucrose fatty acid ester, polyethylene glycol fatty acid ester, and tocopherol-polyethylene glycol-succinate (TPGS).

The total content of the emulsifier in the selfemulsifying composition of the present invention is as defined in claim 1. The total content of the emulsifier is 1 to 29% by weight, preferably 3 to 27% by weight, more preferably 5 to 27% by weight, still more preferably 5 to 24% by weight, and particularly preferably 10 to 20% by weight when the total amount of the self-emulsifying composition is 100% by weight. Alternatively, the total content of the emulsifier is preferably 8 to 27% by weight, and more preferably 10 to 27% by weight. In relation to 100 parts by weight of the ω3 PUFA, the total content of the emulsifier is 5 to 45 parts by weight, preferably 10 to 45 parts by weight, more preferably 15 to 35 parts by weight, and particularly preferably 15 to 20 parts by weight.

The composition of the present invention and the pharmaceutical preparation of the present specification contain a small amount of water. Addition of water to a composition containing a hydrophobic lipid is generally conceived as a loss of compatibility. Presence of water in the composition in itself results in the improved compatibility of the composition, and the use of the polyhydric alcohol and the ethanol becomes unnecessary. In other words, a product having good appearance which is free from the problem of separation or cloudiness of the composition could be produced without using the polyhydric alcohol or the ethanol.

The small amount of water may be added during the preparation of the self-emulsifying composition, and the water in the gelatin capsule film may be transferred to the self-emulsifying composition after the encapsulation of the self-emulsifying composition in the capsule.

In addition, the composition free from the polyhydric alcohol and the ethanol does not cause the capsule to be softened or deformed after the encapsulation, nor has side effects of the ethanol on alcohol intolerance patients taking the composition.

The water is preferably used at an amount of 0.5 to 6% by weight, more preferably 0.5 to 4% by weight, more preferably 0.5 to 3% by weight, and most preferably 1 to 3% by weight when the total amount of the self-emulsifying composition is 100% by weight. Alternatively, the water is preferably contained at an amount of 0.5% by weight or more but less than 3% by weight, and more preferably 0.5% by weight or more but less than 1.5% by weight.

In the present invention, the "lecithin" is a type of glycerophospholipid, and examples include soybean lecithin, zymolytic soybean lecithin, hydrogenated soybean lecithin, egg yolk lecithin, hydrogenated phospholipid, phospholipid from milk, lysolecithin, phosphatidyl choline, and phosphatidyl serine. The preferred are soybean lecithin, zymolytic soybean lecithin, hydrogenated soybean lecithin, and egg yolk lecithin, and the more preferred is soybean lecithin. These may be used alone or in combination of two or more. The term "lecithin" as used in the present invention means all of such glycerophospholipids unless otherwise noted. In the present invention, lecithin is not included in the emulsifier.

Various lecithins are commercially available, and exemplary such products include purified soybean lecithin (Nisshin Oilio), purified egg yolk lecithin (Asahi Kasei Pharma Corporation), and egg yolk lecithin PL-100M (Kewpie Corporation), and use of such products is also possible. Exemplary soybean lecithins include BASIS LP-20B (Nisshin Oil Mills, Ltd.) and Lipoid S45 and S20 (Lipoid), and exemplary zymolytic lecithins include BASIS LP-20E (Nisshin Oil Mills, Ltd.) and Phospholipon RLPC20 (Lipoid). Various such commercially available products may be used in the composition.

The amount of the lecithin added in the self-emulsifying composition of the present invention is as defined in claim 1. The amount of the lecithin added is 3 to 25 parts by weight, preferably 3 to 20 parts by weight, more preferably 3.2 to 17 parts by weight, still more preferably 3.5 to 15 parts by weight, and particularly preferably 3.7 to 17 parts by weight in relation to 100 parts by weight of the ω3 PUFA. Alternatively, the amount of the lecithin added is preferably 3 to 15 parts by weight, more preferably 3 to 12 parts by weight, still more preferably 3 to 10 parts by weight, and most preferably 5 to 10 parts by weight.

In the present invention, the "polyhydric alcohol" is a polyol compound having the structure of a straight chain or cyclic aliphatic hydrocarbon wherein two or more carbon atoms are each substituted with one hydroxy group. Exemplary such polyhydric alcohols include divalent alcohols such as ethylene glycol, propylene glycol, trimethylene glycol, 1,2-butylene glycol, tetramethylene glycol, 1,3-butylene glycol, 2,3-butylene glycol, and pentamethylene glycol; trivalent alcohols such as glycerin, trimethylolpropane, and 1,2,6-hexane triol; and polyhydric alcohol polymers such as diethylene glycol, dipropylene glycol triethylene glycol, polyethylene glycol, polypropylene glycol, and polyglycerin, and the preferred is propylene glycol or glycerin. The glycerin also includes concentrated glycerin. The term "polyhydric alcohol" as used in the present invention means all of such polyol compounds unless otherwise noted.

Content of the polyhydric alcohol in the self-emulsifying composition of the present invention is as defined in claim 1 i.e. an amount that the capsule is not deformed when the composition is filled in the capsule. The content of the polyhydric alcohol in the composition is not more than 4% by weight when the total composition is 100% by weight. Content of the polyhydric alcohol in the composition is up to 4% by weight, preferably up to 3% by weight, more preferably up to 2% by weight, still more preferably up to 1% by weight, and most preferably 0% by weight.

Content of the ethanol in the self-emulsifying composition of the present invention is as defined in claim 1, i.e. an amount that change in the quality is not induced during the encapsulation, distribution, or storage, and denaturing of the capsule content is not induced. Also, the ethanol content does not exceed the daily experientially allowable medical dose. The content of the ethanol in the composition is not more the 4% by weight when the total composition is 100% by weight. Content of the ethanol in the composition is up to 4% by weight, preferably up to 3% by weight, more preferably up to 2% by weight, still more preferably up to 1% by weight, and most preferably 0% by weight.

When the ethanol and the polyhydric alcohol are added in the self-emulsifying composition, the total content of the ethanol and the polyhydric alcohol is preferably not more than 4% by weight when the total amount of the composition is 100% by weight. In the preferred embodiment, the composition does not substantially contain the ethanol or the polyhydric alcohol. Total content of the ethanol and the polyhydric alcohol in the composition is preferably up to 4% by weight, more preferably up to 3% by weight, even more preferably up to 2% by weight, still more preferably up to 1% by weight, and most preferably 0% by weight.

Preferable ethanol concentration may be appropriately determined based on the ω3 PUFA concentration of the self-emulsifying composition and the daily dose of the self-emulsifying composition. When the self-emulsifying composition of the present invention is orally administered at a daily dose of 1800 mg in terms of ω3 PUFA, and a preparation containing the ω3 PUFA, for example, at 75% by weight is prepared, the ethanol dose will not exceed 3.26 mg which is daily maximum dose described in the "Dictionary of Pharmaceutical Additives" when the ethanol concentration is up to 0.135% by weight.

For the self-emulsifying composition of the present invention containing such ω3 PUFA and the emulsifier, the preferred embodiment is the combination containing 1) EPA-E and/or DHA-E, 2) water, 3) a polyoxyethylene sorbitan fatty acid ester as the emulsifier, and 4) lecithin. When the total amount of the self-emulsifying composition is 100% by weight, the EPA-E and/or DHA-E 1) is 70 to 90% by weight, the water 2) is 0.5 to 6% by weight, the emulsifier as defined in claim 1 is 1 to 29% by weight, and the lecithin 4) is 3 to 25 parts by weight in relation to 100 parts by weight of the EPA-E and/or DHA-E. Another preferred embodiment is the combination containing 1) EPA-E and/or DHA-E, 2) water, 3) a polyoxyethylene sorbitan fatty acid ester and 4) polyoxyl castor oil, both as the emulsifier, and 5) lecithin. When the total amount of the self-emulsifying composition is 100% by weight, the EPA-E and/or DHA-E 1) is 70 to 90% by weight, the water 2) is 0.5 to 6% by weight, the emulsifier as defined in claim 1 and the polyoxyl castor oil 4) (excluding the lecithin) is 1 to 29% by weight, and the lecithin 5) is 3 to 25 parts by weight in relation to 100 parts by weight of the EPA-E and/or DHA-E. Another preferred embodiment is the combination containing 1) at least one compound selected from ω3 PUFA and pharmaceutically acceptable salts and esters thereof, 2) water, 3) a polyoxyethylene sorbitan fatty acid ester and polyoxyethylene castor oil, both as the emulsifier, and 4) lecithin. When the total amount of the self-emulsifying composition is 100% by weight, the at least one compound selected from ω3 PUFA and pharmaceutically acceptable salts and esters thereof 1) is 70 to 90% by weight, the water 2) is 0.5 to 6% by weight, the emulsifier including the polyoxyethylene sorbitan fatty acid ester and the polyoxyethylene castor oil 3) is 5 to 24% by weight, where the polyoxyethylene castor oil is up to 120 parts by weight in relation to 100 parts by weight of the polyoxyethylene sorbitan fatty acid ester, and the lecithin 4) is 3 to 25 parts by weight in relation to 100 parts by weight of the at least one compound selected from ω3 PUFA and pharmaceutically acceptable salts and esters thereof.

The self-emulsifying composition of the present invention may be accommodated in a capsule. The capsule selected may be a hard capsule or a soft capsule, and preferably, the capsule used is a soft capsule. The soft capsule is not particularly limited for its shape, and preferably, the soft capsule is a rotary die type soft capsule or a seamless capsule.

In the soft capsule of the present invention, the capsule film is not necessarily limited for its composition, and exemplary main ingredients include gelatin, carageenan, pectin, pullulan, sodium arginate, starch, hypromellose, hydroxypropyl cellulose, and other known ingredients. The preferred is gelatin. The type of gelatin used is not particularly limited, and exemplary gelatins include acid-treated gelatin, alkali-treated gelatin, amphoteric gelatin, chemically modified gelatin, and other known gelatins, which may be used alone or in combination of two or more. The gelatin used is preferably an acid-treated gelatin or alkali-treated gelatin. The source of the gelatin is not necessarily limited, and the gelatin used may be the one from cattle bone, cattle skin, pig bone, pig skin, fish scale, or fish skin, and preferably, the one from cattle bone, cattle skin, pig bone, or pig skin.

The "gelatin" used may be the one normally used in the production of a soft capsule, for example, medical gelatin (gelatin and purified gelatin) defined in The Japanese Pharmacopoeia 16th edition. The gelatin may also be a combination of two or more types. The capsule film may also contain other components such as a plasticizing agent.

The "plasticizing agent" added to the capsule film may be the one normally used in the production of a soft capsule, with preferred examples including a polyhydric alcohol such as glycerin (for example, concentrated glycerin), ethylene glycol, polyethylene glycol, propylene glycol, or polypropylene glycol, and a sugar alcohol such as sorbitol, mannitol, or xylitol. These plasticizing agents may be used in combination of two or more. Particularly preferred are glycerin and sorbitol. Also preferred is a combination of glycerin and sorbitol. In this case, the glycerin and the sorbitol may be used at a weight ratio in the range of 1:5 to 5:1, and more preferably 1:3 to 3:1.

In the soft capsule preparation, and in particular, in the seamless capsule of the present invention, the capsule film solution preferably contains the gelatin and the plasticizing agent at a weight ratio in the range of 10:1 to 1:10, and more preferably of 10:1 to 1:1.

The weight ratio between the capsule film solution and the capsule content is typically 10:1 to 1:10, and preferably 3:1 to 1:10.

If desired, the capsule film may also contain various additives commonly used in the capsule film. Exemplary such additives include amino acids, citric acid, glycerin, sorbitol, trehalose, and other plasticizing agents, antiseptic, dye, titanium oxide, and other colorants, and organic acids.

The composition for the capsule film may be prepared by dissolving gelatin, the plasticizing agent, and the optional additives in water at room temperature or at an elevated temperature.

Preferably, the encapsulated self-emulsifying preparation having the self-emulsifying composition of the present invention encapsulated as its content has high hardness immediately after the production, and this hardness is preferably maintained during the storage. Loss of the hardness is unfavorable in view of the quality because the loss of the hardness does not only result in the deformation but also brittleness and cracking of the capsule and bleeding of the content. Softening of the capsule can be detected by measuring the hardness with a common hardness tester.

The encapsulated self-emulsifying preparation disclosed in the present specification has the hardness immediately after the production of at least 177 N (18 kgf), preferably at least 196 N (20 kgf), and more preferably at least 216 N (22 kgf). It is desirable that the hardness of the preparation does not substantially decrease, or not decrease by 56 N (6 kgf) or more when the preparation is stored in a tightly sealed aluminum package at 40°C for 1 week compared with the hardness immediately after the production. The inventive preparation has a hardness after the storage at 40°C for 1 week of at least 98 N (10 kgf), preferably of at least 147 N (15 kgf), and more preferably of at least 196 N (20 kgf).

When the hardness immediately after the production is 100%, the hardness is maintained after the storage in a tightly sealed aluminum package at 40°C for 1 week at not less than 60%, preferably at not less than 70%, more preferably at not less than 80%, even more preferably at not less than 85%, and most preferably at not less than 90% of that immediately after the production.

The dose and dosage period of the ω3 PUFA used in the self-emulsifying composition of the present invention are made sufficient for realizing the intended action, and can be adequately adjusted depending on the administration route, frequency of administration per day, seriousness of the symptoms, body weight, age, and other factors.

In the case of oral administration, the composition may be administered at a dose in terms of the EPA-E of 0.1 to 5 g/day, preferably 0.2 to 3 g/day, more preferably 0.3 to 3 g/day, and most preferably 0.5 to 3 g/day in 1 to 3 divided doses. However, the entire dose may be administered at once or in several divided doses. The frequency of administration per day is preferably once daily, or twice or thrice daily in divided doses. In the case of once daily administration of, for example, a soft capsule containing 1 g of the EPA-E, 1 to 10 capsules, preferably 1 to 8 capsules, more preferably 1 to 6 capsules, even more preferably 1 to 4 capsules, and still more preferably 1 to 3 capsules may be administered. In addition, a soft capsule containing 1 g of the EPA-E and a soft capsule containing 0.5 g of the EPA-E may be combined to allow the administration of 0.5 g per dose, 1.5 g per dose, 2.5 g per dose, 3.5 g per dose, 4.5 g per dose, or 5.5 g per dose. While meal affects absorption of the EPA-E, and the administration of the EPA-E is preferably conducted during the meal or after the meal, and more preferably immediately after the meal (within 30 minutes after the meal), the self-emulsifying composition of the present invention has excellent absorption under fasting, and therefore, it exhibits the intended effects even when administered at a timing other than during, after, or immediately after the meal, for example, before or immediately before the meal, between meals, or at bedtime; when administered to patients with reduced absorption ability of the intestinal tract (for example, elderly, patients of intestinal disease, patients after intestinal surgery, terminal cancer patients, or patients taking a lipase inhibitor); or when administered at a reduced dose.

The self-emulsifying composition of the present invention is preferably characterized in that it reaches maximum blood concentration in the same or shorter time period after oral administration compared to an ω3 PUFA stock solution. Alternatively, the self-emulsifying composition of the present invention is preferably characterized in that the maximum blood concentration is higher than that of the ω3 PUFA stock solution. Furthermore, the self-emulsifying composition of the present invention is preferably characterized in that blood concentration 2 hours after the administration, and area under blood concentration vs time curve from the administration to 2 hours and/or 72 hours after the administration are equivalent to or higher or larger than those of the ω3 PUFA stock solution. The self-emulsifying composition of the present invention is preferably characterized in that it reaches maximum blood concentration in a shorter time period than the ω3 PUFA; the maximum blood concentration is higher than that of the ω3 PUFA; and blood concentration 2 hours after the administration, and area under blood concentration vs time curve from the administration to 2 hours and/or 72 hours after the administration are all higher or larger than those of the ω3 PUFA stock solution.

The pharmacokinetics described above can be confirmed with animals such as dogs and monkeys but are preferably confirmed by a test with human.

In a pharmacokinetic study in male beagles, when the self-emulsifying composition is orally administered to a male beagle which has been fasted for at least 18 hours at an amount corresponding to 600 mg of the ω3 PUFA, maximum blood ω3 PUFA concentration (corrected by subtracting the blood ω3 concentration before the administration of the composition) is preferably at least 50 µg/mL, more preferably at least 60 µg/mL, and particularly preferably at least 70 µg/mL, and area under the blood ω3 PUFA concentration vs time curve from the administration to 2 hours after the administration is preferably at least 50 µg/mL.hr, more preferably at least 60 µg/mL.hr, and particularly preferably at least 70 µg/mL.hr. A combination of the maximum blood ω3 PUFA concentration and the area under the blood ω3 PUFA concentration vs time curve is preferably at least 50 µg/mL and at least 50 µg/mL.hr, more preferably at least 60 µg/mL and at least 60 µg/mL.hr, and particularly preferably at least 70 µg/mL and at least 70 µg/mL.hr.

In a pharmacokinetic study in male cynomolgus monkeys, when the self-emulsifying composition is orally administered to a male cynomolgus monkey which has been fasted for at least 12 hours at an amount corresponding to 45 mg/kg body weight of the ω3 PUFA, maximum blood ω3 PUFA concentration (corrected by subtracting the blood ω3 concentration before the administration of the composition) is preferably at least 50 µg/mL, and more preferably at least 70 µg/mL, and area under the blood ω3 PUFA concentration vs time curve from the administration to 12 hours after the administration is preferably at least 400 µg/mL.hr, and more preferably at least 500 µg/mL.hr. A combination of the maximum blood ω3 PUFA concentration and the area under the blood ω3 PUFA concentration vs time curve is preferably at least 50 µg/mL and at least 400 µg/mL.hr, and more preferably at least 70 µg/mL and at least 500 µg/mL.hr.

In a pharmacokinetic study in humans, when the self-emulsifying composition is orally administered to a human before the meal at an amount corresponding to 1800 mg of the ω3 PUFA or the EPA, maximum blood ω3 PUFA concentration (corrected by subtracting the blood ω3 concentration before the administration of the composition) is preferably at least 50 µg/mL, more preferably at least 100 µg/mL, even more preferably at least 150 µg/mL, still more preferably at least 200 µg/mL, and most preferably at least 300 µg/mL. Alternatively, the maximum blood ω3 PUFA concentration is preferably 10 to 1000 µg/mL, more preferably 20 to 500 µg/mL, even more preferably 40 to 300 µg/mL, still more preferably 50 to 150 µg/mL, and most preferably 50 to 100 µg/mL. Area under the blood ω3 PUFA concentration vs time curve from the administration to 72 hours after the administration is preferably at least 500 µg/mL.hr, more preferably at least 1000 µg/mL.hr, even more preferably at least 1500 µg/mL.hr, still more preferably at least 2000 µg/mL.hr, and most preferably at least 3000 µg/mL.hr. Alternatively, the area under the blood ω3 PUFA concentration vs time curve is preferably 500 to 4500 µg/mL.hr, more preferably 600 to 3000 µg/mL.hr, even more preferably 700 to 2500 µg/mL.hr, still more preferably 800 to 2000 µg/mL.hr, and most preferably 1000 to 1500 µg/mL.hr. Time-to-maximum plasma concentration is preferably up to 6 hours, more preferably up to 5 hours, even more preferably up to 3 hours, still more preferably up to 1 hour, and most preferably up to 0 hours(less than 1 hour). Alternatively, the time-to-maximum plasma concentration is preferably 0.5 to 10 hours, more preferably 1 to 8 hours, even more preferably 1.5 to 7 hours, still more preferably 2 to 5 hours, and most preferably 2.5 to 4 hours. Elimination half-life in plasma is preferably at least 10 hours, more preferably at least 20 hours, even more preferably at least 30 hours, still more preferably at least 40 hour, and most preferably at least 50 hour. Alternatively, the elimination half-life in plasma is preferably 0 to 150 hours, more preferably 10 to 120 hours, even more preferably 30 to 100 hours, still more preferably 25 to 75 hours, and most preferably 25 to 50 hours.

In a pharmacokinetic study in humans, when the self-emulsifying composition is orally administered to a human before, immediately after, or after the meal at an amount corresponding to 3600 mg of the ω3 PUFA or the EPA, maximum blood ω3 PUFA concentration (corrected by subtracting the blood ω3 concentration before the administration of the composition) is preferably at least 50 µg/mL, more preferably at least 100 µg/mL, even more preferably at least 150 µg/mL, still more preferably at least 200 µg/mL, and most preferably at least 300 µg/mL. Alternatively, the maximum blood ω3 PUFA concentration is preferably 10 to 1000 µg/mL, more preferably 20 to 500 µg/mL, even more preferably 50 to 400 µg/mL, still more preferably 100 to 300 µg/mL, and most preferably 150 to 200 µg/mL. Area under the blood ω3 PUFA concentration vs time curve from the administration to 72 hours after the administration is preferably at least 500 µg/mL.hr, more preferably at least 1000 µg/mL.hr, even more preferably at least 1500 µg/mL.hr, still more preferably at least 2000 µg/mL.hr, and most preferably at least 3000 µg/mL.hr. Alternatively, the area under the blood ω3 PUFA concentration vs time curve is preferably 500 to 5000 µg/mL.hr, more preferably 1000 to 4700 µg/mL.hr, even more preferably 1500 to 4500 µg/mL.hr, still more preferably 2000 to 4000 µg/mL.hr, and most preferably 2500 to 3500 µg/mL.hr. Time-to-maximum plasma concentration is preferably up to 6 hours, more preferably up to 5 hours, even more preferably up to 3 hours, still more preferably up to 1 hour, and most preferably up to 0 hour. Alternatively, the time-to-maximum plasma concentration is preferably 0.5 to 10 hours, more preferably 1 to 8 hours, even more preferably 1.5 to 7 hours, still more preferably 2 to 6 hours, and most preferably 3 to 5 hours. Elimination half-life in plasma is preferably at least 10 hours, more preferably at least 20 hours, even more preferably at least 30 hours, still more preferably at least 40 hour, and most preferably at least 50 hour. Alternatively, the elimination half-life in plasma is preferably 0 to 150 hours, more preferably 10 to 120 hours, even more preferably 30 to 100 hours, still more preferably 25 to 75 hours, and most preferably 25 to 50 hours.

In the case of a pharmacokinetic study in humans, the following ranges may be applied other than the ranges described above. Specifically, when the self-emulsifying composition is orally administered before, immediately after, or after the meal at an amount corresponding to 1800 mg of the EPA, maximum plasma ω3 PUFA concentration (corrected by subtracting the blood ω3 concentration before the administration of the composition) is not particularly limited, and may be in a range selected from, for instance, 10 to 50, 50 to 100, 100 to 150, 150 to 200, 200 to 250, 250 to 300, 300 to 350, 350 to 400, 400 to 450, 450 to 500, 500 to 600, 600 to 700, 700 to 800, 800 to 900, 900 to 1000, 10 to 30, 20 to 40, 30 to 50, 40 to 60, 50 to 70, 60 to 80, 70 to 90, 80 to 100, 90 to 110, 100 to 120, 110 to 130, 120 to 140, 130 to 150, 140 to 160, 150 to 170, 160 to 180, 170 to 190, 180 to 200, 190 to 210, 200 to 220, 220 to 240, 240 to 260, 260 to 280, 280 to 300, 10 to 20, 15 to 25, 20 to 30, 25 to 35, 30 to 40, 35 to 45, 40 to 50, 45 to 55, 50 to 55, 53 to 58, 55 to 60, 58 to 63, 60 to 65, 63 to 68, 65 to 70, 68 to 73, 70 to 75, 73 to 78, 75 to 80, 78 to 83, 80 to 85, 83 to 88, 85 to 90, 88 to 93, 90 to 95, 93 to 98, 95 to 100, 98 to 103, 100 to 105, 103 to 108, 105 to 110, 108 to 113, 110 to 115, 113 to 118, 115 to 120, 118 to 123, 120 to 125, 123 to 128, 125 to 130, 128 to 133, 130 to 135, 133 to 138, 135 to 140, 138 to 143, 140 to 145, 143 to 148, 145 to 150, 150 to 160, 155 to 165, 160 to 170, 165 to 175, 170 to 180, 175 to 185, 180 to 190, 185 to 195, 190 to 200, 195 to 205, 200 to 210, 205 to 215, 210 to 220, 215 to 225, 220 to 230, 225 to 235, 230 to 240, 235 to 245, and 240 to 250 µg/mL, and when the self-emulsifying composition is administered before, immediately after, or after the meal at an amount corresponding to 3600 mg of the EPA, the maximum plasma ω3 PUFA concentration may be in a range selected from, for instance, 10 to 50, 50 to 100, 100 to 150, 150 to 200, 200 to 250, 250 to 300 300 to 350, 350 to 400, 400 to 450, 450 to 500, 500 to 600, 600 to 700, 700 to 800, 800 to 900, 900 to 1000, 10 to 30, 20 to 40, 30 to 50, 40 to 60, 50 to 70, 60 to 80, 70 to 90, 80 to 100, 90 to 110, 100 to 120, 110 to 130, 120 to 140, 130 to 150, 140 to 160, 150 to 170, 160 to 180, 170 to 190, 180 to 200, 190 to 210, 200 to 220, 220 to 240, 240 to 260, 260 to 280, 280 to 300, 10 to 20, 15 to 25, 20 to 30, 25 to 35, 30 to 40, 35 to 45, 40 to 50, 45 to 55, 50 to 55, 53 to 58, 55 to 60, 58 to 63, 60 to 65, 63 to 68, 65 to 70, 68 to 73, 70 to 75, 73 to 78, 75 to 80, 78 to 83, 80 to 85, 83 to 88, 85 to 90, 88 to 93, 90 to 95, 93 to 98, 95 to 100, 98 to 103, 100 to 105, 103 to 108, 105 to 110, 108 to 113, 110 to 115, 113 to 118, 115 to 120, 118 to 123, 120 to 125, 123 to 128, 125 to 130, 128 to 133, 130 to 135, 133 to 138, 135 to 140, 138 to 143, 140 to 145, 143 to 148, 145 to 150, 150 to 160, 155 to 165, 160 to 170, 165 to 175, 170 to 180, 175 to 185, 180 to 190, 185 to 195, 190 to 200, 195 to 205, 200 to 210, 205 to 215, 210 to 220, 215 to 225, 220 to 230, 225 to 235, 230 to 240, 235 to 245, and 240 to 250 µg/mL.

When the self-emulsifying composition is administered before, immediately after, or after the meal at an amount corresponding to 1800 mg of the EPA, area under the blood ω3 PUFA concentration vs time curve from the administration to 72 hours after the administration may be in a range selected from, for instance, 500 to 1500, 1000 to 2000, 1500 to 2500, 2000 to 3000, 2500 to 3500, 3000 to 4000, 500 to 1000, 750 to 1250, 1000 to 1500, 1250 to 1750, 1500 to 2000, 1750 to 2250, 2000 to 2500, 2250 to 2750, 2500 to 3000, 2750 to 3250, 3000 to 3500, 3250 to 3750, 3500 to 4000, 3750 to 4250, 4000 to 4500, 4250 to 4750, 4500 to 5000, 500 to 700, 600 to 800, 700 to 900, 800 to 1000, 900 to 1100, 1000 to 1200, 1100 to 1300, 1200 to 1400, 1300 to 1500, 1400 to 1600, 1500 to 1700, 1600 to 1800, 1700 to 1900, 1800 to 2000, 1900 to 2100, 2000 to 2200, 2100 to 2300, 2200 to 2400, 2300 to 2500, 2400 to 2600, 2500 to 2700, 2600 to 2800, 2700 to 2900, 2800 to 3000, 2900 to 3100, 3000 to 3200, 3100 to 3300, 3200 to 3400, 3300 to 3500, 3400 to 3600, 3500 to 3700, 3600 to 3800, 3700 to 3900, 3800 to 4000, 3900 to 4100, 4000 to 4200, 4100 to 4300, 4200 to 4400, 4300 to 4500, 500 to 600, 550 to 650, 600 to 700, 650 to 750, 700 to 800, 750 to 850, 800 to 900, 850 to 950, 900 to 1000, 950 to 1050, 1000 to 1100, 1050 to 1150, 1100 to 1200, 1150 to 1250, 1200 to 1300, 1250 to 1350, 1300 to 1400, 1350 to 1450, 1400 to 1500, 1450 to 1550, 1500 to 1600, 1550 to 1650, 1600 to 1700, 1650 to 1750, 1700 to 1800, 1750 to 1850, 1800 to 1900, 1850 to 1950, 1900 to 2000, 1950 to 2050, 2000 to 2100, 2050 to 2150, 2100 to 2200, 2150 to 2250, 2200 to 2300, 2250 to 2350, 2300 to 2400, 2350 to 2450, 2400 to 2500, 2450 to 2550, 2500 to 2600, 2550 to 2650, 2600 to 2700, 2650 to 2750, 2700 to 2800, 2750 to 2850, 2800 to 2900, 2850 to 2950, 2900 to 3000, 2950 to 3050, 3000 to 3100, 3150 to 3250, 3200 to 3300, 3250 to 3350, 3300 to 3400, 3350 to 3450, 3400 to 3500, 3500 to 3600, 3600 to 3700, 3700 to 3800, 3800 to 3900, 3900 to 4000, 4000 to 4100, 4100 to 4200, 4200 to 4300, 4300 to 4400, and 4400 to 4500 µg/mL.hr, and when the self-emulsifying composition is administered before, immediately after, or after the meal at an amount corresponding to 3600 mg of the EPA, the area under the blood ω3 PUFA concentration vs time curve may be in a range selected from, for instance, 500 to 1500, 1000 to 2000, 1500 to 2500, 2000 to 3000, 2500 to 3500, 3000 to 4000, 500 to 1000, 750 to 1250, 1000 to 1500, 1250 to 1750, 1500 to 2000, 1750 to 2250, 2000 to 2500, 2250 to 2750, 2500 to 3000, 2750 to 3250, 3000 to 3500, 3250 to 3750, 3500 to 4000, 3750 to 4250, 4000 to 4500, 4250 to 4750, 4500 to 5000, 500 to 700, 600 to 800, 700 to 900, 800 to 1000, 900 to 1100, 1000 to 1200, 1100 to 1300, 1200 to 1400, 1300 to 1500, 1400 to 1600, 1500 to 1700, 1600 to 1800, 1700 to 1900, 1800 to 2000, 1900 to 2100, 2000 to 2200, 2100 to 2300, 2200 to 2400, 2300 to 2500, 2400 to 2600, 2500 to 2700, 2600 to 2800, 2700 to 2900, 2800 to 3000, 2900 to 3100, 3000 to 3200, 3100 to 3300, 3200 to 3400, 3300 to 3500, 3400 to 3600, 3500 to 3700, 3600 to 3800, 3700 to 3900, 3800 to 4000, 3900 to 4100, 4000 to 4200, 4100 to 4300, 4200 to 4400, 4300 to 4500, 500 to 600, 550 to 650, 600 to 700, 650 to 750, 700 to 800, 750 to 850, 800 to 900, 850 to 950, 900 to 1000, 950 to 1050, 1000 to 1100, 1050 to 1150, 1100 to 1200, 1150 to 1250, 1200 to 1300, 1250 to 1350, 1300 to 1400, 1350 to 1450, 1400 to 1500, 1450 to 1550, 1500 to 1600, 1550 to 1650, 1600 to 1700, 1650 to 1750, 1700 to 1800, 1750 to 1850, 1800 to 1900, 1850 to 1950, 1900 to 2000, 1950 to 2050, 2000 to 2100, 2050 to 2150, 2100 to 2200, 2150 to 2250, 2200 to 2300, 2250 to 2350, 2300 to 2400, 2350 to 2450, 2400 to 2500, 2450 to 2550, 2500 to 2600, 2550 to 2650, 2600 to 2700, 2650 to 2750, 2700 to 2800, 2750 to 2850, 2800 to 2900, 2850 to 2950, 2900 to 3000, 2950 to 3050, 3000 to 3100, 3150 to 3250, 3200 to 3300, 3250 to 3350, 3300 to 3400, 3350 to 3450, 3400 to 3500, 3500 to 3600, 3600 to 3700, 3700 to 3800, 3800 to 3900, 3900 to 4000, 4000 to 4100, 4100 to 4200, 4200 to 4300, 4300 to 4400, and 4400 to 4500 µg/mL.hr.

When the self-emulsifying composition is administered before, immediately after, or after the meal at an amount corresponding from 1800 mg to 3600 mg of the EPA, time-to-maximum plasma concentration may be in a range selected from, for instance, 0 to 2, 1 to 3, 2 to 4, 3 to 5, 4 to 6, 5 to 7, 6 to 8, 7 to 9, 8 to 10, 0 to 1, 0.5 to 1.5, 1 to 2, 1.5 to 2.5, 2 to 3, 2.5 to 3.5, 3 to 4, 3.5 to 4.5, 4 to 5, 4.5 to 5.5, 5 to 6, 5.5 to 6.5, 6 to 7, 6.5 to 7.5, 7 to 8, 7.5 to 8.5, 8 to 9, 8.5 to 9.5, 9 to 10, 0 to 0.5, 0.3 to 0.8, 0.5 to 1, 0.8 to 1.3, 1 to 1.5, 1.3 to 1.8, 1.5 to 2, 1.8 to 2.3, 2 to 2.5, 2.3 to 2.8, 2.5 to 3, 2.8 to 3.3, 3 to 3.5, 3.3 to 3.8, 3.5 to 4, 3.8 to 4.3, 4 to 4.5, 4.3 to 4.8, 4.5 to 5, 4.8 to 5.3, 5 to 5.5, 5.3 to 5.8, 5.5 to 6, 5.8 to 6.3, 6 to 6.5, 6.3 to 6.8, 6.5 to 7, 6.8 to 7.3, 7 to 7.5, 7.3 to 7.8, 7.5 to 8, 7.8 to 8.3, 8 to 8.5, 8.3 to 8.8, 8.5 to 9, 8.8 to 9.3, 9 to 9.5, 9.3 to 9.8, and 9.5 to 10 hours.

When the self-emulsifying composition is administered before, immediately after, or after the meal at an amount corresponding from 1800 mg to 3600 mg of the EPA, elimination half-life in plasma may be in a range selected from, for instance, 0 to 50, 25 to 75, 50 to 100, 75 to 125, 100 to 150, 125 to 175, 150 to 200, 0 to 20, 10 to 30, 20 to 40, 30 to 50, 40 to 60, 50 to 70, 60 to 80, 70 to 90, 80 to 100, 90 to 110, 100 to 120, 110 to 130, 120 to 140, 130 to 150, 0 to 10, 5 to 15, 10 to 20, 15 to 25, 20 to 30, 25 to 35, 30 to 40, 35 to 45, 40 to 50, 45 to 55, 50 to 60, 55 to 65, 60 to 70, 65 to 75, 70 to 80, 75 to 85, 80 to 90, 85 to 95, 90 to 100, 95 to 105, 100 to 110, 105 to 115, and 110 to 120 hours.

The present invention may be specified by combining two or more selected from the maximum plasma ω3 PUFA concentration, the area under the blood ω3 PUFA concentration vs time curve from the administration to 72 hours after the administration, the time-to-maximum plasma concentration, and the elimination half-life in plasma as described above.

The self-emulsifying composition of the present invention may also contain additives such as an emulsification aid, stabilizer, antiseptic, surfactant, and antioxidant. Exemplary emulsification aids include fatty acids containing 12 to 22 carbon atoms such as stearic acid, oleic acid, linoleic acid, palmitic acid, linolenic acid, and myristic acid and their salts. Exemplary stabilizers include phosphatidic acid, ascorbic acid, glycerin, and cetanols. Exemplary antiseptics include ethyl paraoxybenzoate and propyl paraoxybenzoate. Exemplary surfactants include sucrose fatty acid esters, sorbitan fatty acid esters, glycerin fatty acid esters, polyoxyethylene sorbitan fatty acid esters, polyoxyethylene alkyl ethers, polyoxyethylene fatty acid esters, polyoxyethylene alkyl phenyl ethers, and polyoxyethylene polyoxypropylene alkyl ethers. Exemplary antioxidants include oil-soluble antioxidants such as butylated hydroxy toluene, butylated hydroxy anisole, propyl gallate, propyl gallate, pharmaceutically acceptable quinone, astaxanthin, and α-tocopherol.

In addition, an adequate carrier or mediater, a colorant, a flavor, and optionally, a vegetable oil and an additive such as non-toxic organic solvent or non-toxic solubilizing agent, emulsifier, suspending agent (for example, Tween 80 and gum arabic solution), isotonic agent, pH adjusting agent, stabilizer, corrective, flavoring agent, preservative, antioxidant, or absorption promoter commonly used in the art may be adequately combined with the inventive composition to prepare an appropriate pharmaceutical preparation.

More specifically, since the ω3 PUFA is highly unsaturated, effective amount of an oil-soluble antioxidant, for example, at least one member selected from butylated hydroxytoluene, butylated hydroxyanisole, propyl gallate, propyl gallate, pharmaceutically acceptable quinone, astaxanthin, and α-tocopherol is preferably incorporated in the composition.

Since the self-emulsifying composition of the present invention is also used for pharmaceutical application, it preferably has good appearance, self-emulsifying property, composition dispersibility, emulsion stability, and storage stability. The appearance of the self-emulsifying composition is such that the composition is not separated, clouded, solidified, or precipitated, but transparent. The composition having poor appearance may be pharmaceutically unsuitable, and such composition may be insufficient in required performance such as self-emulsifying property.

With regard to the storage temperature, the self-emulsifying composition and the preparation prepared by encapsulating such composition is preferably transparent at both low temperature and high temperature in view of its use in cold district or hot environment.

In the case of the self-emulsifying composition having good self-emulsifying property, good dispersibility of the composition, and high emulsion stability, the composition rapidly disperses upon contact with water to form a microemulsion having adequate emulsion droplet diameter. Absorbability of an oil such as EPA-E is related to the size of the emulsion droplet diameter, and degree of the absorbability upon administration to the animal can be estimated by measuring the emulsion droplet diameter.

In the present invention, the "mean droplet diameter" is the value of volume mean diameter among droplets of the emulsified composition measured by using a particle size analyzer (for example, Nanotorac manufactured by Nikkiso Co., Ltd.) with water being used for the dispersion medium according to standard measurement method (for example, set zero time of 30 seconds, measurement time of 30 seconds, average of three measurements). The mean droplet diameter when the self-emulsifying composition of the present invention is dispersed in water or the like is not particularly limited as long as it is up to 2 µm, and the product has good emulsion dispersibility, good emulsion stability, or good absorbability, and the mean droplet diameter is typically up to 1.5 µm, more preferably up to 1.0 µm, still more preferably up to 0.5 µm, and most preferably up to 0.3 µm.

The self-emulsifying composition of the present invention may be used by combining with a second effective component. The second effective component may be any component adequately selected depending on the intended type and severity of the disease as long as it does not adversely affect the merits of the ω3 PUFAs. Exemplary such second effective components include therapeutic agents for hyperlipidemia, antihypertensives, antidiabetics, antioxidants, blood flow improving agents, bile acid derivatives, therapeutic agents for NAFLD and NASH, and progression inhibitors and therapeutic agents for dementia.

In preferred second effective components, exemplary therapeutic agents for hyperlipidemia include polyenephosphatidylcholine, unsaponified soybean oil (soysterol), gamma-oryzanol, riboflavine butyrate, dextran sulfate sodium sulfur 18, pantethine, and elastase, as well as statins such as pravastatin, simvastatin, atorvastatin, fluvastatin, pitavastatin, rosuvastatin, and cerivastatin, fibrate drugs such as simfibrate, clofibrate, clinofibrate, bezafibrate, and fenofibrate, lipase inhibitors such as orlistat, and cetilistat, resins such as colestyramine, and colestimide, and ezetimibe.

Exemplary antihypertensives include angiotensin II receptor antagonists such as irbesartan, olmesartan medoxomil, candesartan cilexetil, telmisartan, valsartan, and losartan potassium, angiotensin converting enzyme inhibitors such as alacepril, imidapril hydrochloride, enalapril maleate, captopril, quinapril hydrochloride, cilazapril hydrate, temocapril hydrochloride, delapril hydrochloride, trandolapril, benazepril hydrochloride, perindopril, and lisinopril hydrate, calcium antagonists such as azelnidipine, amlodipine besylate, aranidipine, efonidipine hydrochloride, cilnidipine, nicardipine hydrochloride, nifedipine, nimodipine, nitrendipine, nilvadipine, barnidipine hydrochloride, felodipine, benidipine, and manidipine, alpha blockers such as tolazoline, and phentolamine, beta blockers such as atenolol, metoprolol, acebutolol, propranolol, pindolol, carvedilol, and labetalol hydrochloride, alpha receptor agonists such as clonidine, and methyldopa, and diuretics such as eplerenone, hydrochlorothiazide, and furosemide.

Exemplary antidiabetics include alpha-glucosidase inhibitors such as acarbose, voglibose, and miglitol, sulfonylurea hypoglycemic agents such as gliclazide, glibenclamide, glimepiride, and tolbutamide, short acting insulin secretagogues such as nateglinide, and mitiglinide, biguanide hypoglycemic agents such as metformin hydrochloride, and buformin hydrochloride, dipeptidyl phosphatase 4 inhibitors such as sitagliptin, vildagliptin, alogliptin, linagliptin, teneligliptin, anagliptin, and saxagliptin, thiazolidine drugs such as pioglitazone hydrochloride, and rosiglitazone maleate, glucagon-like peptide 1 derivative drugs such as exenatide, and liraglutide, and sodium-glucose co-transporter 2 inhibitors such as ipragliflozin, dapagliflozin, luseogliflozin, tofogliflozin, canagliflozin, and empagliflozin.

Exemplary antioxidants include vitamins such as ascorbic acid (vitamin C), tocopherol (vitamin E), and tocopherol nicotinate, n-acetylcysteine, and probucol.

Exemplary blood flow improving agents include cilostazol, ticlopidine hydrochloride, alprostadil, limaprost, beraprost sodium, sarpogrelate hydrochloride, argatroban, naftidrofuryl, isoxsuprine hydrochloride, batroxobin, dihydroergotoxine mesilate, tolazoline hydrochloride, hepronicate, and Simotsuto extract.

Exemplary bile acid derivatives include ursodeoxycholic acid, chenodeoxycholic acid, bile powder, deoxycholic acid, cholic acid, bile extract, bear bile, oriental bezoar, and dehydrocholic acid. In addition, preferred examples of the bile acid derivative include biotin (vitamin B7), cyanocobalamin (vitamin B12), pantothenic acid (vitamin B5), folic acid (vitamin B9), thiamine (vitamin B1), vitamin A, vitamin D, vitamin K, tyrosine, pyridoxine (vitamin B6), branched chain amino acids such as leucine, isoleucine, and valine, calcium, iron, zinc, copper, and magnesium, as well as components of food for specified health uses and food with nutrient function claims such as soybean protein, chitosan, low-molecular-weight sodium alginate, dietary fiber derived from psyllium seed husks, soy peptide with bound phospholipid, plant sterol ester, plant stanol ester, diacylglycerol, globin proteolysis product, and tea catechin.

Exemplary therapeutic agents for NAFLD and NASH include statin drugs such as pravastatin, simvastatin, atorvastatin, fluvastatin, pitavastatin, rosuvastatin, and cerivastatin, angiotensin II receptor antagonists such as irbesartan, olmesartan medoxomil, candesartan cilexetil, telmisartan, valsartan, and losartan potassium, biguanide hypoglycemic agents such as metformin hydrochloride, and buformin hydrochloride, and thiazolidine drugs such as pioglitazone hydrochloride, and rosiglitazone maleate as described above, as well as farnesoid X receptor (hereinafter abbreviated as FXR) ligands such as aspirin, ursodeoxycholic acid, chenodeoxycholic acid, and obeticholic acid.

Exemplary progression inhibitors and therapeutic agents for dementia include acetylcholinesterase inhibitors such as donepezil hydrochloride, and galantamine hydrobromide, NMDA receptor inhibitor such as memantine hydrochloride, antiplatelet agents such as aspirin, clopidogrel sulfate, cilostazol, and ticlopidine hydrochloride, and factor Xa inhibitors such as rivaroxaban, and apixaban. In addition, the therapeutic agents for hyperlipidemia, the antihypertensives, the antidiabetics, the antioxidants, the blood flow improving agents, and the like as described above may also be used as the progression inhibitors and therapeutic agents for dementia.

To realize pharmacological actions of the ω3 PUFA, the self-emulsifying composition of the present invention preferably has at least one merit selected from good appearance, good self-emulsifying property, high composition dispersibility, high emulsion stability, high storage stability (including the stability at low and high temperatures), high absorbability, in particular high absorbability and high absorption speed under fasting conditions, and convenience in taking the preparation or compliance.

The self-emulsifying composition of the present invention is well adapted for use as a therapeutic agent for treating various diseases of animals, mammals in particular, that is to say, is usable as, for instance, therapeutic agent for dyslipidemias (hypercholesterolemia, high LDL cholesterolemia, high non-HDL cholesterolemia, high VLDL cholesterolemia, low HDL cholesterolemia, hypertriglyceridemia, hyperapobetalipoproteinemia, apo A-I hypolipoproteinemia, and the like), therapeutic agent for postprandial hypertriglyceridemia, anti-arteriosclerotic, platelet aggregation suppressant, therapeutic agent for peripheral circulatory insufficiency, agent for preventing occurrence of cardiovascular events, therapeutic agent for inflammatory diseases (NAFLD, NASH, and the like), progression inhibitor and therapeutic agent for dementia (Alzheimer-type dementia, cerebrovascular dementia, mixed type dementia, and the like), anticancer agent, and therapeutic agent for central diseases (clinical depression, depressive state, obsessive-compulsive disorder, social anxiety disorder, panic disorder, and the like). In the treatment of the diseases described above, the frequency of administration per day is not particularly limited and is preferably once daily, or twice or thrice daily in divided doses, more preferably once or twice daily, and even more preferably once daily.

The self-emulsifying composition of the present invention is particularly effective in improving or treating or secondarily preventing dyslipidemia and postprandial hypertriglyceridemia and in preventing progress to metabolic syndrome, cardiocerebrovascular event, and ulcer and gangrene in limbs and periphery. Exemplary mammals include human, domestic animals such as cattle, horse, and pig, and companion animals such as dog, cat, rabbit, rat, and mouse, and the preferred is human. More specifically, the self-emulsifying composition of the present invention is anticipated to show ameliorating or therapeutic effects for dyslipidemia and postprandial hypertriglyceridemia in patients with dyslipidemia suffering from increase in the blood lipid, exhibiting insulin resistance or suffering from increase in the blood pressure, such as metabolic syndrome patients.

### EXAMPLES

Next, the present invention is described in further detail by referring to the following Examples and Comparative Examples which by no means limit the scope of the invention.

### Example 1

0.09 g of water, 0.53 g of polyoxyethylene (20) sorbitan oleate, 0.39 g of soybean lecithin, and 4.0 g of EPA-E were weighed, sealed, and mixed while heating to about 70°C to thereby prepare the self-emulsifying composition. The self-emulsifying composition was sealed by purging with nitrogen, and stored at room temperature until the evaluation was conducted. Formulation of the self-emulsifying composition is shown in Table 1.

### Examples 2 to 11 and Comparative Examples 1 and 2

The self-emulsifying compositions of Examples 2 to 11 and the compositions of Comparative Examples 1 and 2 were prepared and stored by repeating the method of Example 1 so that the compositional ratios were as shown in Table 1. Formulations of the self-emulsifying compositions and the compositions are shown in Table 1.

### Comparative Examples 3 and 4

The compositions of Comparative Examples 3 and 4 were prepared and stored by repeating the method of Example 1 so that the compositional ratios were as shown in Table 1. Formulation of the composition is shown in Table 1.

The self-emulsifying compositions and the compositions of Comparative Examples as produced by the method as described above were each encapsulated in the soft capsule containing gelatin as its main component.

### Test Example 1 <Evaluation of appearance>

After producing the self-emulsifying compositions and compositions of the Comparative Examples by the production method as described above, the compositions were allowed to stand, and after about 1 hour, their appearance was evaluated. When the composition was homogeneous due to the good compatibility, the composition was evaluated "transparent." The composition was evaluated "separated" when the separation was observed, and "cloudy" when opacity was observed.

The results are shown in Table 1.

### Test Example 2 <Evaluation of self-emulsifying property>

The self-emulsifying compositions and compositions of the Comparative Examples produced by the production method as described above were evaluated for self-emulsifying property by adding 10 µL of each composition dropwise to 5 mL of purified water or first solution in the dissolution test of Japanese Pharmacopeia at 37°C in the test tube. The composition which spontaneously emulsified just by the dropwise addition was evaluated "good", and the case which did not become an emulsion just by the dropwise addition was evaluated "poor." The composition was then lightly stirred under consistent condition, and the property was evaluated. With regard to the dispersibility of the composition, the composition was evaluated "good" when dispersed and "poor" when the composition was partly left undispersed as a mass. With regard to the emulsion stability, the composition was evaluated "good" when no oil separation was observed, and "poor" when oil separation was observed. It is to be noted that the compositions which were not evaluated as "transparent" were not evaluated since those having non-homogeneous composition were conceived to be inadequate for the evaluation.

The results are shown in Table 1.

### Test Example 3 <Evaluation of emulsion droplet diameter>

Mean droplet diameter (volume mean diameter) of the emulsified material was evaluated by using about 1.5 mL of the emulsified composition obtained in Test Example 2 using a particle size analyzer (Nanotorac, manufactured by Nikkiso Co., Ltd.) with water being used for the dispersion medium.

### Test Example 4 <Evaluation of the appearance after storage under severe conditions>

The compositions which were evaluated "transparent" or "cloudy" in Test Example 1 were allowed to stand and stored overnight (about 12 hours) at 5°C or 40°C before their appearance was evaluated. When the composition was homogeneous due to the good compatibility, the composition was evaluated "transparent". The composition was evaluated "separated" when the separation was observed, and "cloudy" when opacity was observed.

The results are shown in Table 1.

### Test Example 5 <Pharmacokinetics in beagles>

The composition or the capsule prepared was orally administered to 6 male beagles (at the age of 2 to 6 years with the body weight of 8 to 13 kg, 3 Marshall beagles and 3 Nosan beagles) under fasting conditions, and blood EPA concentration was evaluated. The test animals were fasted 18 hours or more before the administration, and each animal was administered with the composition at an amount corresponding to 600 mg of the EPA-E. Blood was collected before the administration, and 0.5, 1, 1.5, 2, 3, 4, 6, 8, and 24 hours after the administration, and plasma was separated to measure plasma EPA concentration by LC/MS/MS (a method of separating a sample by liquid chromatography and separating the resultant components by mass spectrometry for measurement). The control group was administered with the EPA-E stock solution encapsulated in a capsule.

Table 1 shows maximum blood concentration (Cmax) calculated from the test results, the area under the blood concentration vs time curve from 0 to 2 hours (AUC₀₋₂), and the area under the blood concentration vs time curve from 0 to 24 hours (AUC₀₋₂₄). In the calculation of each parameter, the value was corrected by subtracting the blood EPA concentration before the administration of the composition.

### Test Example 6 <Pharmacokinetics in cynomolgus monkeys>

The composition of Example 14 is orally administered to 6 cynomolgus monkeys (at the age of 2 to 5 years with the body weight of 2.70 to 4.65 kg, from Hamri Co., Ltd.) under fasting conditions, and blood EPA concentration is evaluated. The test animals are fasted 12 hours or more before the administration, and each animal is administered with the self-emulsifying composition at an amount corresponding to 45 mg/kg of the EPA-E. The control group is administered with the EPA-E stock solution encapsulated in a capsule. Blood is collected before the administration, and 1, 2, 4, 6, 8, 10, 12, 24, 48, and 72 hours after the administration, and plasma is separated to measure plasma EPA concentration by LC/MS/MS. The test results are used to calculate maximum blood concentration (Cmax), the area under the blood concentration vs time curve from 0 to 12 hours (AUC₀₋₁₂), and the area under the blood concentration vs time curve from 0 to 72 hours (AUC₀₋₇₂). In the calculation of each parameter, the value is corrected by subtracting the blood EPA concentration before the administration of the composition.

In the animals administered with the composition of Example 14, increase in the blood concentration parameters such as Cmax and AUC₀₋₁₂ is confirmed compared to the control group. In other words, it is confirmed that, when the self-emulsifying composition of Example 14 is administered, amount of the EPA absorbed increases, and also, EPA absorption is rapid after the oral administration.

### Test Example 7 <Appearance of the capsule>

The soft capsules obtained in the Examples were visually inspected for the color and shape of the capsule and the property of the capsule content after the completion of filling and drying.

The capsules with change in the "color", distortion, depression, or the like in the "shape", and cloudiness, separation, or the like in the "property of the capsule content" were evaluated "poor", and the capsules without such problems were evaluated "normal."

The results are shown in Table 1. In the Table below, "-" means that the corresponding component was not added or that the corresponding item was not measured.

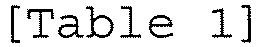

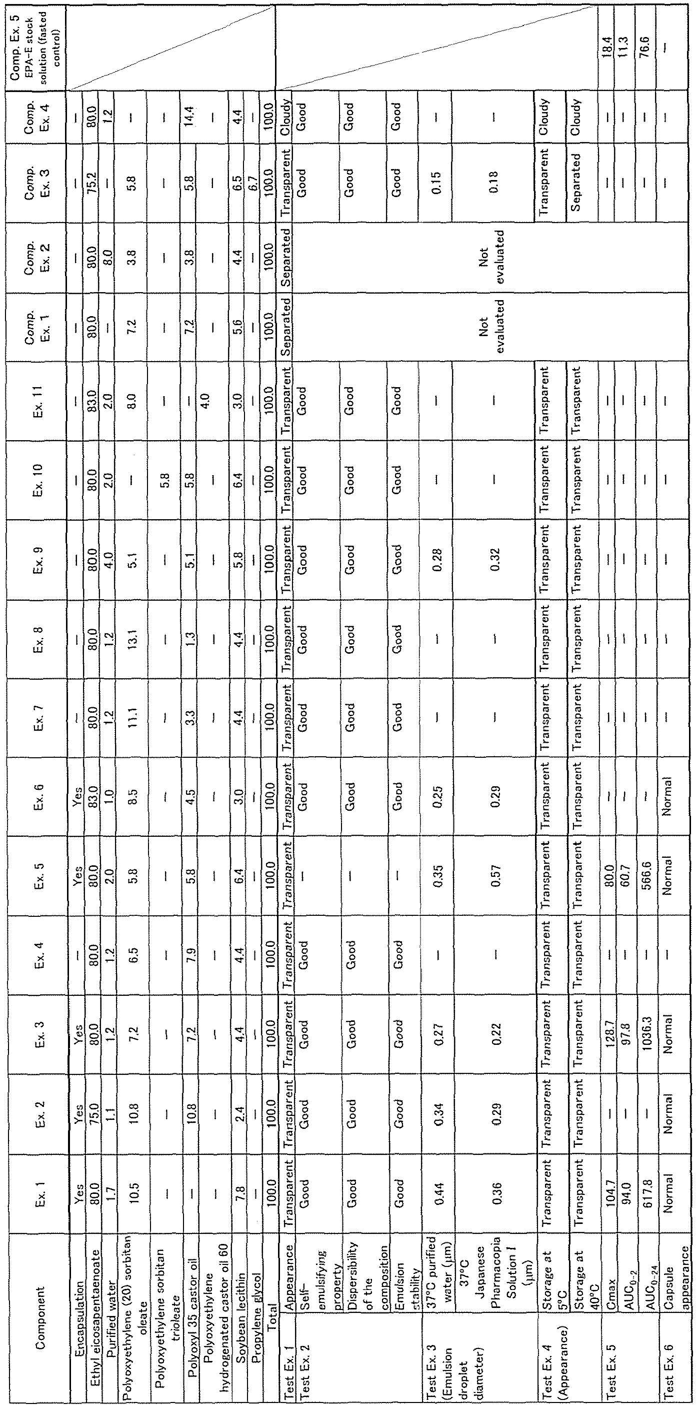

Example 1 is the composition containing polyoxyethylene sorbitan fatty acid ester as the only emulsifier together with certain amounts of lecithin and water, and as shown in Table 1, this composition had good appearance as well as excellent self-emulsifying property. This result indicates that the merits of the present invention are realized by the composition containing lecithin wherein the polyoxyethylene sorbitan fatty acid ester is the only emulsifier.

Examples 2 to 10 are compositions containing polyoxyethylene castor oil as an additional emulsifier. These compositions also exhibited good appearance as well as excellent self-emulsifying property as shown in Table 1.

Example 11 is the composition containing polyoxyethylene hydrogenated castor oil as an additional emulsifier. This composition also exhibited good appearance as well as excellent self-emulsifying property as shown in Table 1.

Comparative Example 1 is a composition not containing the water, and this composition became separated. Comparative Example 2 is a composition containing 8% by weight of water, and this composition also became separated.

In the present invention, water was used instead of adding the ethanol or the polyhydric alcohol to thereby improve the compatibility of the composition. The composition became separated due to inadequate compatibility when no water was used, while the composition still separated even with the use of water when the amount of water was too much in relation to the amount of the composition. In the meanwhile, the separation did not occur in Examples 1 to 6 containing 1 to 4% by weight of water. These results indicate that presence of a certain amount (approximately 0.5 to 6% by weight) of water is important for the good appearance.

Comparative Example 3 is a composition not containing water but containing polyhydric alcohol. As in the case of Example 1, this composition had good appearance as well as good self-emulsifying property.

However, the composition of Comparative Example 3 was separated after overnight storage at 40°C. This demonstrates importance of adding a particular amount (approximately 0.5 to 6% by weight) of water to the composition in view of improving the appearance and the like.

Comparative Example 4 is a composition containing polyoxyethylene castor oil as an emulsifier and not containing polyoxyethylene sorbitan fatty acid ester. This composition had cloudy appearance.

This demonstrates importance of adding the polyoxyethylene sorbitan fatty acid ester as an emulsifier in view of improving the appearance.

Examples 1, 3, and 5 show the kinetics when the self-emulsifying composition was administered to fasted animals.

In the animals having these self-emulsifying compositions administered under fasting, Cmax and AUC₀₋₂ values which are the parameters of the absorption speed were significantly higher than the control group (fasted) as administered with the EPA-E stock solution. More specifically, it was confirmed that, when the self-emulsifying compositions of the Examples were administered, amount of the EPA absorbed until 24 hours after the oral administration increased, and also, EPA absorption was rapid especially after the oral administration compared to the control group. Accordingly, the self-emulsifying composition of the present invention would be a candidate for the self-emulsifying preparation capable of rapidly and markedly increasing the blood EPA concentration with rapid and effective pharmacological action even if taken under pre-meal, pre-sleeping, or other fasting conditions.

### Self-emulsifying capsule preparations of Examples 2-1 and 2-2 and capsule preparation of Comparative Example 2-3

The self-emulsifying compositions and the composition of Comparative Example 2-3 were prepared and stored by repeating the procedure of Example 1 so that the compositional ratios were as shown in Table 2. The formulations of the self-emulsifying compositions are shown in Table 2.

375 mg of the self-emulsifying composition was filled in the capsule in Examples 2-1 and 2-2, and 441 mg of the self-emulsifying composition was filled in the capsule in Comparative Example 2-3 (300 mg of EPA-E in both cases). In each case, the soft gelatin capsule having the content filled therein was produced by rotary method. Deformation of the capsule film was not noticed for the self-emulsifying capsule preparation prepared by this method.

The compositional ratios of the encapsulated contents are shown in Table 2.

### Test Example 8 <Hardness of the capsules

The capsule preparations of Examples 2-1 and 2-2 and Comparative Example 2-3 were measured in hardness. The preparations after storing at 40°C and a relative humidity of 75% for 1, 2, and 4 weeks were also measured in hardness.

The results at the initial stage and the results after the storage at 40°C for 1, 2, and 4 weeks are shown in Table 2. The preparation at the initial stage is the preparation which has been stored at room temperature after its preparation until the evaluation of the hardness. The capsules are not affected by the moisture since each preparation was placed in an aluminum sealed package and stored at 40°C.

**[Table 2]**

| Component | | Ex. 2-1 wt% | Ex. 2-2 wt% | Ex. 2-3 wt% |
|---|---|---|---|---|
| Ethyl eicosapentaenoate | | 80.0 | 80.0 | 68.0 |
| Purified water | | 2.0 | 1.8 | - |
| Polyoxyethylene (20) sorbitan oleate | | 5.8 | 5.8 | 7.1 |
| Polyoxyl 35 castor oil | | 5.8 | 5.8 | 7.1 |
| Soybean lecithin | | 6.4 | 6.4 | 9.5 |
| Zymolytic lecithin | | - | 0.2 | - |
| Tocopherol | | - | - | - |
| Sodium erythorbate | | - | - | - |
| Propylene glycol | | - | - | 8.3 |
| Total | | 100.0 | 100.0 | 100.0 |
| Test Example 7 Hardness (kgf) | Initial stage | 28.9 | 24.7 | 15.7 |
| | 40°C, 1 week | 25.5 | 22.3 | 9.1 |
| | 40°C, 2 weeks | 24.5 | 20.5 | 8.9 |
| | 40°C, 4 weeks | 27.4 | 22.3 | 8.1 |

Examples 2-1 and 2-2 are preparations produced by encapsulating the self-emulsifying composition of the 196 N present invention. These capsules had a hardness of (20 kgf) or higher. On the other hand, the composition of Comparative Example 2-3 contained a large amount (8.3% by weight) of propylene glycol (a polyhydric alcohol), and the hardness of the capsule preparation was already inferior compared to Examples at the initial stage. When the hardness was evaluated after storing in sealed environment at 40°C for 1 to 4 weeks, substantially no change was noticed in Examples 2-1 and 2-2, while, in Comparative Example 2-3, the hardness declined to 57% of the initial hardness in 1 week, and further declined with time.

### Industrial Applicability

The self-emulsifying composition of the present invention is excellent in at least one of the compatibility (appearance), self-emulsifying property, composition dispersibility, emulsion stability, and absorbability, and it, as being absorbed rapidly even if taken before the meal, suppresses increase of serum TG after the meal. The self-emulsifying composition of the present invention is useful for incorporating in various foods, or as food for special dietary uses, food with health claims (food for specified health use or food with nutrient function claims), health food (supplement), or a pharmaceutical product.

The self-emulsifying composition of the present invention has no or low content of the polyhydric alcohol, and therefore, the composition is free from the problem of softening and deformation of the capsule during the distribution or storage caused by the polyhydric alcohol. In other words, the self-emulsifying composition of the present invention is associated with reduced risk of quality change.
The self-emulsifying composition of the present invention has the quality as a pharmaceutical product which may be stored in a cold or hot location since the composition does not become cloudy or separated even if stored in low or high temperature environment.

## Claims

1. A self-emulsifying composition comprising, when the total amount of the self-emulsifying composition is 100% by weight,
a) 70 to 90% by weight of at least one compound selected from the group consisting of an w3 PUFA, its pharmaceutically acceptable salt, and its ester,
b) 0.5 to 6% by weight of water,
c) 1 to 29% by weight of an emulsifier which is a polyoxyethylene sorbitan fatty acid ester, and
d) 3 to 25 parts by weight of lecithin in relation to 100 parts by weight of the w3 polyunsaturated fatty acid, its pharmaceutically acceptable salt, and its ester,
wherein
e) content of ethanol is up to 4% by weight of total amount of the composition, and
f) content of polyhydric alcohol is up to 4% by weight of total amount of the composition.

2. The self-emulsifying composition according to claim 1 wherein the emulsifier further contains a polyoxyethylene hydrogenated castor oil and/or a polyoxyethylene castor oil.

3. The self-emulsifying composition according to claim 1 wherein the emulsifier contains a polyoxyethylene sorbitan fatty acid ester and a polyoxyethylene castor oil.

4. The self-emulsifying composition according to any one of claims 1 to 3 wherein the polyhydric alcohol is propylene glycol or glycerin.

5. The self-emulsifying composition according to any one of claims 1 to 4 wherein content of the ethanol is up to 1% by weight of total amount of the composition.

6. The self-emulsifying composition according to any one of claims 1 to 5 wherein content of the polyhydric alcohol is up to 1% by weight of total amount of the composition.

7. The self-emulsifying composition according to any one of claims 1 to 6 comprising, when the total amount of the self-emulsifying composition is 100% by weight,
a) 70 to 90% by weight of at least one compound selected from the group consisting of an w3 polyunsaturated fatty acid, its pharmaceutically acceptable salt, and its ester,
b) 0.5 to 6% by weight of water,
c) 5 to 24% by weight of an emulsifier which includes a polyoxyethylene sorbitan fatty acid ester and a polyoxyethylene castor oil, and
d) 3 to 40 parts by weight of lecithin in relation to 100 parts by weight of the w3 polyunsaturated fatty acid, its pharmaceutically acceptable salt, and its ester,
wherein
e) content of the polyoxyethylene castor oil is up to 120 parts by weight in relation to 100 parts by weight of the polyoxyethylene sorbitan fatty acid ester,
f) content of ethanol is up to 4% by weight of total amount of the composition, and
g) content of polyhydric alcohol is up to 4% by weight of total amount of the composition.

## Patentansprüche

1. Selbstemulgierende Zusammensetzung, umfassend, wenn die Gesamtmenge der selbstemulgierenden Zusammensetzung 100 Gew.-% beträgt,
a) 70 bis 90 Gew.-% mindestens einer Verbindung, ausgewählt aus der Gruppe bestehend aus einer w3-PUFA, deren pharmazeutisch verträglichem Salz und deren Ester,
b) 0,5 bis 6 Gew.-% Wasser,
c) 1 bis 29 Gew.-% eines Emulgators, der ein Polyoxyethylensorbitan-Fettsäureester ist, und
d) 3 bis 25 Gewichtsteile Lecithin, bezogen auf 100 Gewichtsteile der mehrfach ungesättigten w3-Fettsäure, deren pharmazeutisch verträglichem Salz und deren Ester,
wobei
e) der Ethanolgehalt bis zu 4 Gew.-% der Gesamtmenge der Zusammensetzung beträgt und
f) der Gehalt an mehrwertigem Alkohol bis zu 4 Gew.-% der Gesamtmenge der Zusammensetzung beträgt.

2. Die selbstemulgierende Zusammensetzung nach Anspruch 1, wobei der Emulgator zusätzlich ein hydriertes Polyoxyethylen-Rizinusöl und/oder ein Polyoxyethylen-Rizinusöl enthält.

3. Die selbstemulgierende Zusammensetzung nach Anspruch 1, wobei der Emulgator einen Polyoxyethylensorbitan-Fettsäureester und ein Polyoxyethylen-Rizinusöl enthält.

4. Die selbstemulgierende Zusammensetzung nach einem der Ansprüche 1 bis 3, wobei der mehrwertige Alkohol Propylenglykol oder Glycerin ist.

5. Die selbstemulgierende Zusammensetzung nach einem der Ansprüche 1 bis 4, wobei der Ethanolgehalt bis zu 1 Gew.-% der Gesamtmenge der Zusammensetzung beträgt.

6. Die selbstemulgierende Zusammensetzung nach einem der Ansprüche 1 bis 5, wobei der Gehalt an mehrwertigem Alkohol bis zu 1 Gew.-% der Gesamtmenge der Zusammensetzung beträgt.

7. Die selbstemulgierende Zusammensetzung nach einem der Ansprüche 1 bis 6, umfassend, wenn die Gesamtmenge der selbstemulgierenden Zusammensetzung 100 Gew.-% beträgt
a) 70 bis 90 Gew.-% mindestens einer Verbindung, ausgewählt aus der Gruppe bestehend aus einer mehrfach ungesättigten w3-Fettsäure, deren pharmazeutisch verträglichem Salz und deren Ester,
b) 0,5 bis 6 Gew.-% Wasser,
c) 5 bis 24 Gew.-% eines Emulgators, der einen Polyoxyethylensorbitan-Fettsäureester und ein Polyoxyethylen-Rizinusöl enthält, und
d) 3 bis 40 Gewichtsteile Lecithin, bezogen auf 100 Gewichtsteile der mehrfach ungesättigten w3-Fettsäure, deren pharmazeutisch verträglichem Salz und deren Ester,
wobei
e) der Gehalt an Polyoxyethylen-Rizinusöl bis zu 120 Gewichtsteile, bezogen auf 100 Gewichtsteile des Polyoxyethylensorbitan-Fettsäureesters, beträgt,
f) der Gehalt an Ethanol bis zu 4 Gew.-% der Gesamtmenge der Zusammensetzung beträgt und
g) der Gehalt an mehrwertigem Alkohol bis zu 4 Gew.-% der Gesamtmenge der Zusammensetzung beträgt.

## Revendications

1. Composition auto-émulsifiante comprenant, lorsque la quantité totale de la composition auto-émulsifiante est de 100 % en poids,
a) de 70 à 90 % en poids d'au moins un composé sélectionné dans le groupe constitué d'ω3 PUFA, son sel pharmaceutiquement acceptable, et son ester,
b) de 0,5 à 6 % en poids d'eau,
c) de 1 à 29 % en poids d'un agent émulsifiant qui est un ester d'acide gras de polyoxyéthylène sorbitan, et
d) de 3 à 25 parties en poids de lécithine en relation à 100 parties en poids de l'acide gras ω3 polyinsaturé, son sel pharmaceutiquement acceptable, et son ester,
dans laquelle
e) une teneur de l'éthanol atteint jusqu'à 4 % en poids de la quantité totale de la composition, et
f) une teneur de l'alcool polyhydroxylé atteint jusqu'à 4 % en poids d'une quantité totale de la composition.

2. Composition auto-émulsifiante selon la revendication 1, dans laquelle l'agent émulsifiant contient en outre de l'huile de ricin hydrogénée de polyoxyéthylène et/ou de l'huile de ricin de polyoxyéthylène.

3. Composition auto-émulsifiante selon la revendication 1, dans laquelle l'agent émulsifiant contient un ester d'acide gras de polyoxyéthylène sorbitan et une huile de ricin de polyoxyéthylène.

4. Composition auto-émulsifiante selon l'une quelconque des revendications 1 à 3, dans laquelle l'alcool polyhydroxylé est le propylène glycol ou la glycérine.

5. Composition auto-émulsifiante selon l'une quelconque des revendications 1 à 4, dans laquelle la teneur de l'éthanol atteint jusqu'à 1 % en poids de la quantité totale de la composition.

6. Composition auto-émulsifiante selon l'une quelconque des revendications 1 à 5, dans laquelle une teneur de l'alcool polyhydroxylé atteint jusqu'à 1 % en poids d'une quantité totale de la composition.

7. Composition auto-émulsifiante selon l'une quelconque des revendications 1 à 6, comprenant, lorsque la quantité totale de la composition auto-émulsifiante est de 100 % en poids,
a) de 70 à 90 % en poids d'au moins un composé sélectionné dans le groupe constitué d'un acide gras ω3 polyinsaturé, son sel pharmaceutiquement acceptable, et son ester,
b) de 0,5 à 6 % en poids d'eau,
c) de 5 à 24 % en poids d'un agent émulsifiant qui inclut un ester d'acide gras de polyoxyéthylène sorbitan et une huile de ricin de polyoxyéthylène, et
d) de 3 à 40 parties en poids de lécithine en relation à 100 parties en poids de l'acide gras w3 polyinsaturé, son sel pharmaceutiquement acceptable, et son ester,
dans laquelle
e) une teneur de l'huile de ricin de polyoxyéthylène atteint jusqu'à 120 parties en poids en relation à 100 parties en poids d'ester d'acide gras de polyoxyéthylène sorbitan,
f) une teneur de l'éthanol atteint jusqu'à 4 % en poids d'une quantité totale de la composition, et
g) une teneur de l'alcool polyhydroxylé atteint jusqu'à 4 % en poids d'une quantité totale de la composition.
